# EUROPEAN PATENT SPECIFICATION

(11) **EP 4 232 050 B1**
(45) Date of publication and mention of the grant of the patent: **16.09.2026**
(21) Application number: 21887384.2
(22) Date of filing: 26.10.2021
(51) Int. Cl.: A61K 35/12, A61L 27/36, A61L 27/58

(54) **PREPARATION AND USE OF TISSUE MATRIX DERIVED POWDER**
HERSTELLUNG UND VERWENDUNG VON PULVER AUS GEWEBEMATRIX
PRÉPARATION ET UTILISATION DE POUDRE DÉRIVÉE DE MATRICE TISSULAIRE

(30) Priority: 26.10.2020 US 202063105726 P; 26.10.2020 WO PCT/US2020/057431; 25.01.2021 US 202163141338 P
(43) Date of publication of application: 30.08.2023
(73) Proprietor: Jilun Sports Technology Inc., Newport Beach CA 92662 (US)
(72) Inventor: JING, Di, Menlo Park, California 94025 (US); WANG, Xiuyu, Menlo Park, California 94025 (US); HUANG, Zhijun, Menlo Park, California 94025 (US); WANG, Mingxiang, Menlo Park, California 94025 (US); RYZHUK, Volodymyr, Menlo Park, California 94025 (US); GAO, Wenting, Menlo Park, California 94025 (US)
(74) Representative: Mollekopf, Gerd Willi
(86) International application number: PCT/US2021/056724
(87) International publication number: WO 2022/093877

(56) References cited:
- WO-A1-2021/081540
- CN-A- 104 225 667
- CN-A- 110 051 884
- CN-A- 110 051 884
- US-A- 3 318 774
- US-A1- 2011 189 140
- US-A1- 2011 189 140
- US-A1- 2015 037 436
- US-A1- 2019 070 336
- US-A1- 2019 070 336
- US-A1- 2020 030 247
- ANONYMOUS: "Cryogenic grinding - Wikipedia", 11 March 2018 (2018-03-11), pages 1 - 3, XP055815293, Retrieved from the Internet <URL:https://en.wikipedia.org/w/index.php?title=Cryogenic_grinding&oldid=829912867> [retrieved on 20210617]

## Description

### CROSS-REFERENCE TO RELATED APPLICATIONS

### TECHNICAL FIELD

The application is in the field of tissue matrix derived powders, for example the preparation and use of a soluble, injectable, micronized and lyophilized decellularized tissue matrix derived powder (TH Powder) that may be optimized and used for various therapeutic purposes.

### BACKGROUND

Therapeutic biomaterials have been made from human and animal tissue. These biomaterials may be beneficial because they have natural bioactive constituents that may play a role in reducing inflammation, promoting healing and providing other benefits.

The human cells, tissues, and cellular and tissue-based products (HCT/Ps) are processed by minimal manipulations of the tissue ("Regulatory Considerations for Human Cells, Tissues, and Cellular and Tissue-Based Products: Minimal Manipulation and Homologous Use" by the Food and Drug Administration). For example, see U.S. Application Pat. Pub US20140147511A1 Tseng et al., which involves simple and minimal manipulations including obtaining tissue, freezing, lyophilizing and grinding the lyophilized tissue to powder. However, the minimally manipulated HCT/Ps have risks in transmitting viruses and triggering the immune response.

The devitalized tissue derived hydrogels, for example, see U.S. Application Pat. Pub US20080181967A1 Badylak et al., are provided as a sterile hydrogel with only one chosen concentration, such as 1mg/mL, 3mg/mL, 4mg/mL, 5mg/mL, 6mg/mL, 10mg/mL or 20mg/mL. Each concentration leads to an independent product, which requires a separate FDA regulatory clearance or approval. This type of devitalized tissue derived hydrogels become very thick and almost unflowable in the concentration of 20mg/mL. Moreover, the hydrogels may lack long shelf-life (such as two years and above). And it is also difficult for a liquid to maintain product stability, maintain terminal sterility and minimize spontaneous tissue separation and collagen polymerization and solidification (gelation).

In other hydrogel preparation methods, such as frozen or freeze-dried devitalized tissue derived hydrogel, which produce sponge-like structures, for example, U.S. Application Pat. Pub US 2018/0155678A1 Francis et al., US Application Pat. Pub US10213526B2 Badylak et al. and Chinese Patent Application Pat Pub CN 104225667B Chao et al., require a considerable time to rehydrate upon the point of use, such as 24, 36, 48 hours or above, often produce a heterogeneous solution, and may require stirring rod or pipetting to speed up rehydration. In a clinical setting with a demand to ensure efficiency and sterility, a lengthy and/or complex rehydration process is impractical and increases the risk of contaminating the product.

US 2011/189140 A1 discloses compositions comprising decellularized cardiac extracellular matrix and therapeutic uses thereof. Methods of preparing cardiomyocyte culture surfaces and culturing cells with absorbed decellularized cardiac extracellular matrix are provided.

### SUMMARY

The invention is defined in the independent claims. Particular embodiments are set out in the dependent claims.

Improved methods of preparing the soluble, injectable, micronized, and/or lyophilized tissue matrix derived powder (TH Powder) from biological materials are disclosed. These methods allow for manipulation and control of a variety of characteristics of a resulting material, such as a dry and/or a dehydrated tissue powder. As discussed elsewhere herein, preparation steps may be configured to generate the TH Powder having characteristics favorable for specific therapeutic uses.

In some embodiments, the preparation of the TH Powder involves obtaining tissue and maintaining a low initial bioburden by immediately immersing the collected cleaned fresh tissue in antifungal, antiviral, and/or antimicrobial disinfectant solutions followed by freezing of the tissue. For example, peracetic acid is one of the most versatile disinfectants and presents no harm to the environment. Applying peracetic acid during the process of collecting and obtaining tissue presents no harm to the animals, the breeding farms, and the cold chain delivery process. As microorganisms grow very rapidly on the fresh mammalian tissue, such initial treatment (e.g., washing with peracetic acid) may have a significant benefit in maintaining a low bioburden.

In some embodiments, the preparation of the TH Powder involves viral inactivation methods that meet the standards of the US FDA and the US Department of Agriculture (USDA). These viral inactivation methods allow international and domestic shipping and transportation without transmitting human or animal derived viruses. Specific examples of such viral inactivation methods are discussed further herein.

In some embodiments, the preparation of the TH Powder involves a novel decellularization process to produce tissue extracellular matrix. Such a decellularization process efficiently rids the native cells and genetic materials such as DNA and RNA from the tissue, while maintaining desirable bioactive compounds.

In some embodiments, the preparation of the TH Powder involves Electronic Beam (EB) sterilization under temperature controlled conditions. For example, freezing and/or packaging the TH Powder with dry ice for at least 12 hours prior to EB irradiation. This temperature controlled sterilization process maintains desirable bioactive compounds of the TH Powder, and prevents elevated temperatures that can otherwise result from EB irradiation. Further, novel carton box packaging system is disclosed. This packaging system aids in placing TH Powder products to maintain a narrow range in distance between the TH Powder and an EB source. Such placement helps narrow the distribution in electron beam dosage received by the TH Powder. For example, by disposing TH Powder with limited height, relative to an EB source, can reduce the dosage range for batch products from 15 kGy (minimum) to 30 kGy (maximum) dose to 15 kGy (minimum) to 25 kGy (maximum) dose.

In some embodiments, the preparation of the TH Powder involves irradiation Gamma irradiation sterilization. In some embodiments, which are not according to the invention as claimed, the preparation of the TH Powder involves application of Ethylene Oxide (ETO) to tissue at low temperatures. ETO sterilization process temperatures may range from 22 Degrees Celsius to 40 Degrees Celsius, or at dry ice temperatures.

Both EB and Gamma irradiation result in a greater solubility, flowability and injectability of the rehydrated TH Powder compared to ETO sterilization. When irradiated at a dose 15kGy and above, both EB and Gamma irradiation results in a highly soluble, flowable and injectable liquid derived from the rehydrated TH Powder. TH Powder sterilized using EB or Gamma irradiation does not result in a product that will polymerize and/or solidify (e.g., reproducible gelation) at body temperature. In contrast, TH Powder sterilized using low-temperature ETO sterilization can result in rehydrated products that will polymerize and/or solidify (reproducible gelation) at body temperatures. However ETO sterilization typically results in rehydrated products having lower solubility, flowability (more viscous) and injectability relative to similar products sterilized using EB or Gamma irradiation.

Thus, based on the results derived from the sterilization methods above, one can choose a particular sterilization method to generate a product having characteristics applicable for a specific clinical need. Some embodiments include using a combination of sterilization techniques to achieve a product having characteristics between those achievable using any one technique. Any combination of the discussed herein may be used.

Preparation of TH Powder involves two or more rounds of lyophilization and two or more rounds of cryogrinding of decellularized tissue, for example, placenta tissue, amniotic tissue, pericardial tissue, epithelium tissue, to produce a dry powder product (TH Powder). Appropriate selection of lyophilization and/or cryogrinding results in a TH Powder having long shelf-life such as 3 years, 4 years, or 5 years and above, ease of shipping, transportation and storage in all kinds of conditions including air, ground and marine in ambient temperatures. Compared to previously cited the tissue derived hydrogels, the TH Powder allows for maintenance of terminal sterility (e.g., SAL (Sterility Assurance Level) 10-6) and avoids separation of tissue and buffer solutions. Compared to simply frozen or freeze-dried devitalized tissue derived hydrogels that produce sponge-like structures and require a long period of time and/or a complicated process to rehydrate, the 2^{nd} lyophilization and the 2^{nd} cryogrinding of the novel process to produce the TH Powder result in a desirable powder particle size distribution and prompt (nearly instant) rehydration at the time of use. The rehydration can be simple and nearly instant, for example, TH Power can simply be poured into a mixing bottle, saline or other solutions added to the mixing bottle, the mixture shaken well, and then be ready to use.

Cryogrinding, a kind of grinding at low temperatures, prevents destruction of desirable bioactive components of the devitalized tissue. The lyophilization and cryogrinding methods disclosed herein result in highly soluble TH Powder, which not only results in nearly instant and easy rehydration upon the time of use, but also results in a high concentration of the reunified hydrogel derived from the rehydrated TH Powder. In various embodiments, the concentration (as used herein, concentration may mean either this mg of powder per ml of solvent or mass/volume (density) of the resulting solution) of the hydrogel, in various embodiments, to at least 30mg/mL, 35mg/mL, 40mg/mL, 45mg/mL, 50mg/mL, 55mg/mL, 60mg/mL or higher, while maintaining a desirable viscosity (flowability) and injectability. In comparison, traditionally prepared liquid devitalized tissue hydrogels, for example, U.S. Application Pat. Pub. US 20180100139A1 Ryzhuk et al, are only capable of achieving relatively low concentration such as 20mg/mL. Clinicians can rehydrate the TH Powder and achieve various concentrations for clinical needs.

In some embodiments of the invention, a novel cryogrinding treatment system is disclosed. This system results in a desirable particle size distribution that leads to an excellent solubility, flowability and injectability of rehydrated TH Powder. The particle size distribution within the TH Powder can be controlled by grinding conditions and used to control the rate of solubility, flowability, injectability and polymerization/solidification. The smaller particle sizes result in a faster and more evenly mixing during rehydration, allowing for a uniform appearance and avoiding the formation of visible lumps in tissue material suspension, and also allowing a shorter time of polymerization/solidification. Rehydration containers may be a glass vial, a test tube, a plastic spray bottle, a plastic bottle, an eye dropper bottle, a vaginal/anorectal applicator, and the like. The rehydration is optionally nearly instant after several shakes of the rehydration containers. Potentially there may be several small lumps present if the concentration of the TH Powder is high, however, those lumps typically dissolve within a couple of hours after re-shaking the containers. The small particle size enables the use of the rehydrated TH Powder in a spray system, in a drug carrier system, and/or in an injection system that uses small size needles and probes between 22 and 34 Gauge.

Highly concentrated rehydrated TH Powder, such as 20, 30, 40, 50 and even 60 mg/mL, is characterized by higher viscosity, stronger adhesiveness, and shorter polymerization and solidification (reproducible gelation) time compared to the low concentrations, such as 1, 3, 5, 8, 10 mg/mL. Moreover, the smaller particle size of the TH Powder results in a shorter polymerization and solidification (reproducible gelation) time (within about 3-5 min or less than 7 min at normal body temperature). Compared to traditionally prepared liquid tissue derived hydrogels and/or sponge-like structures of the lyophized tissue derived hydrogels that obtain a polymerization and solidification time within roughly 20-30 min or longer normal body temperature. This feature, of various embodiments of the new TH Powder, representing a great improvement in usability.

Various embodiments of the invention disclose a treatment system including a composition comprising the TH Powder having a particle size of D50 (µm) less than 200 µm, wherein the tissue is ground in, optionally multiple, cryogrinding steps at a temperature less than 0, -10, -20 or -30 Degrees Celsius (or any range therebetween), resulting in the TH Powder having particle diameters (D50) less than 200, 100, 50, 30 or 20 µm (See Table 2).

Various embodiments include methods of manufacturing TH Powder for various delivery systems, and methods of treatment. The methods can include, for example, retrieving and cleaning tissue, immersing tissue in antiviral antimicrobial disinfectant solutions such as roughly 0.01% - 1% peracetic acid for 2 hours to reduce the initial bioburden, freezing, shipping tissue in cold chain delivery in ^{~}-10 Degrees Celsius or lower, viral inactivation, washing and preparing tissue, multiple freeze-thaw cycles, decellularization (chemical alteration and physical alteration), 1^{st} lyophilization, 1^{st} cryogrinding to produce a micronized powder having 50% particle diameter sizes optionally in the range of 200 µm or less, digestion and solubilization, neutralization and polymerization, optionally add mixtures such as therapeutic compounds to produce a therapeutic material or add preservatives to produce a multi-use product, and 2^{nd} lyophilization, optionally add mixtures to grind with the lyophilized material, 2^{nd} cryogrinding to produce a micronized powder having 50% particle diameter sizes less than 200 µm, select the particle sizes, packaging and terminal sterilization. The added mixtures can be included in dry powder of the TH Powder. Or, the liquid added mixtures can directly mix with the TH Powder during rehydration, to form a gel, a paste, a liquid, a spray, a drop, a hydrogel and/or the like.

The therapeutic material may be configured for use in a wide variety of clinical applications, including any of those discussed herein. For example, the treatment composition may be used to reduce the growth of undesirable tissue and adhesions (e.g., fibrosis or angiogenesis), to reduce inflammation, to reduce neovascularization, to deliver therapeutic compounds (e.g., antibiotics, bevacizumab or ranibizumab), to treat ocular injuries, to treat intravitreal disease, to treat burns, and/or the like.

Various embodiments of the invention include methods of applying, dissolving and mixing of the TH Powder. The TH Powder may be configured to rehydrate in a glass vial, a plastic spray bottle, a plastic container bottle, a plastic eye dropper bottle, or a vaginal or anorectal applicator; to produce a paste, a liquid, a spray, a drop or a hydrogel upon addition of a solution such as purified water, normal saline, phosphate-buffered saline (PBS), sterile irrigating balanced salt solution (BSS), other bio-compatible salt solutions that may contain sodium chloride and/or potassium chloride, and/or other liquid pharmaceutical drugs or therapeutic agents.

Various embodiments of the invention include a treatment system comprising the TH Powder and a rehydration solution such as purified water, normal saline, PBS, BSS, other biocompatible salt solutions, other pharmaceutical drugs, and other additions that may contain hyaluronic acid (HA), polyethylene glycol (PEG), sodium carboxymethyl cellulose (CMC-Na), hydroxyethylcellulose (HEC), polysaccharides and chitosan, and/or the like, to increase the solubility, viscosity, adhesiveness, shorten the polymerization and solidification time, increase the weight and density, and even prolong biodegradation rate of the rehydrated TH Powder. Various preservatives including potassium sorbate and polyquaternium-1 for multi-use clinical applications may be included in the treatment system.

The TH Powder may be used in a wide range of therapeutic applications, which include dermal, intradermal and subdermal applications, oral and esophageal applications, orthopedic applications, dental applications, neurological applications, obstetric and gynecological applications, ophthalmic applications, vaginal, anorectal, urinary or gastrointestinal tract applications, surgical applications, and/or the like. Further examples of the clinical applications of the TH Powder are discussed elsewhere herein. Various embodiments of the invention include a method of treatment of any one of the ailments discussed herein using the dry or rehydrated TH Powder discussed herein, wherein the ailment is, for example, joint injury or joint degradation, in-stent restenosis, striae gravidarum and striae distensae, many retinal diseases including retinal tears, retinal detachments, macular holes, ocular surface wounds, dry eye, intraocular injury, age-related macular degeneration (AMD), regeneration of retinal pigment epithelium (RPE) cells, diabetic retinopathy and retinal vein occlusion, many ocular surface diseases including corneal/ocular surface wounds, ulcerations and dry eye, gastric reflux injury, mucosal tissue wounds including vaginal or anorectal trauma, oral disorders, esophageal disorders, ureteral irritations, inflammatory bowel disease, Crohn's Disease, ulcerative colitis, radiation proctitis, skin conditions such as chronic and acute skin wounds, rashes, eczema, dermatitis, radiation therapy damage, acne, wrinkle elimination, hydration, skin toning (brown spots, hyperpigmentation and skin redness minimization), skin filling, skin volumizing, and/or the like.

### BRIEF DESCRIPTION OF THE DRAWINGS

FIG. 1 illustrates a methods of manufacturing TH Powder, according to various embodiments of the invention.
FIG. 2 illustrates novel grinding methods, according to various embodiments of the invention.
FIG. 3 illustrates a novel cryogenic grinding system, according to various embodiments of the invention. This system includes Grinding Balls 310, a Nylon Grinding Tank 320, a Refrigerant Cycling System 330 and a Temperature Controllable Grinding Tank 340 according to various embodiments of the invention. The refrigerant is optionally supplied using a refrigerant compressor, silicon transfer tubing and suitable temperature control electronics.
FIG. 4 illustrates a novel carton packaging system of the TH Powder for Electronic Beam (EB) irradiation, according to various embodiments of the invention.
FIG. 5 illustrates DNA content of native tissue and the decellularized tissue processed by the exemplary decellularization methods (Method 1 and Method 2) , according to various embodiments of the invention. *p < 0.05, ***p < 0.001.
FIGs. 6A-6C illustrate use of the TH Powder as evaluated in a mouse xenograft model of breast cancer (4T1) to determine effect on tumor growth, according to various embodiments of the invention.
FIG. 7A illustrates the porous structure of TH Powder, and FIG. 7B illustrates the amount of Recombinant Epidermal Growth Factor, human (hEGF) released in the rehydrated TH Powder as a carrier measured by ELISA analysis, according to various embodiments of the invention.
FIGs. 8A-8B illustrate use of TH Powder evaluated in an in vitro study to determine effect on growth and regeneration of cartilage cells.
FIGs. 9A-9C illustrates use of TH Powder to restore endometrial glandular cell number and reduce fibrosis following injury in a rabbit IUA model, according to various embodiments of the invention.
FIGs. 10A-10C Illustrate use of TH Powder for management of ocular surface wounds following injuries and eases dry eye symptoms in a rat corneal alkali burn model, according to various embodiments of the invention.
FIG. 11 illustrates the ophthalmic ocular surface wound treatment composition kit, according to various embodiments of the invention. The kit comprises a glass vial containing the TH Powder, a dropper bottle and a funnel.
FIGs. 12A-12D illustrate evaluation of TH Powder *in vitro* to determine the viability and anti-angiogenic effect on human retinal pigment epithelial (RPE) cells, according to various embodiments of the invention.
FIG. 13 illustrates the ophthalmic intravitreal treatment composition kit, according to various embodiments of the invention.
FIG. 14 illustrates clinical case study in cancer patients with radiation induced proctitis, according to various embodiments of the invention.
FIG. 15 illustrates clinical case study in cancer patients with radiation induced vaginitis, according to various embodiments of the invention.
FIG. 16 illustrates the vaginal and anorectal wound care treatment composition kit, according to various embodiments of the invention.
FIG. 17 illustrates the skin and oral wound care treatment composition kit, according to various embodiments of the invention.
FIGs. 18A-18C illustrate clinical case studies of patients experiencing chemoradiotherapy dermatitis and using the TH Powder for the management of dermatitis and pain, according to various embodiments of the invention.
FIG. 19 illustrates a clinical case study of a patient experiencing chemoradiotherapy oral mucositis and using the TH Powder for the management of mucositis and pain, according to various embodiments of the invention.
FIG. 20 illustrates clinical case study in cancer patients with radiation induced dermatitis, according to various embodiments of the invention.
FIG. 21 illustrates clinical case study in cancer patients with radiation induced oral mucositis/stomatitis, according to various embodiments of the invention.
FIGs. 22A and 22B illustrate clinical case studies in patients with acne vulgaris, according to various embodiments of the invention.
FIG. 23 illustrates clinical case studies in patients with striae gravidarum, according to various embodiments of the invention.
FIGs. 24A and 25B illustrate a 33 subject (compared to the baseline), single center, randomized clinical trial to determine TH Powder efficacy in 11 clinical claims in moisturizing, anti-aging, improving elasticity and anti-wrinkle, according to various embodiments of the invention.

### DETAILED DESCRIPTION

The TH Powder includes porcine placenta tissue. Alternatively, but not according to the invention claimed, the materials may comprise any combination of human or animal tissues, for example and without limitation, placenta, amnion, chorion, umbilical cord, embryotic tissue, ocular tissue, lymph node tissue, neural tissue, skin, dermis, urinary bladder, small intestine, mesothelium, pericardium, heart valve, fascia lata, liver, lung, heart, adipose, skeletal, blood vessel, nerve conduits, cartilage, breast, colon, or other tissues and organs, stem cells, enzymes, proteins, hormones, bacteria, yeasts, algae, and/or the like. The one or more optional components may include, for example, trace elements, an antimetabolite agent, an antifungal agent, a pain reliever, muscle cells, differentiated stem cells, skin cells, nerve cells, immunological cells, vitamins, viruses, biological compositions, cells, cross-linking materials, hydrogel matrix structures, viscosity control compounds, cross-linking compounds, pharmaceuticals, anti-inflammatory agents, antibodies, T-cells, vaccines, immune system repressors or activators, antibiotics, antiviral agents, enzymes, peptides bacteriophage, thickener, buffer, salt, fat (e.g., omega-3s), natural oils, aloe, mineral oil, antioxidants, coloring agents, flavoring agents, cosmetics, fibrin, stem cell scaffolding, moisturizers, sun screen, tea extracts, vitamin C, hyaluronic acid, lactic acid, alpha- or beta-hydroxy acids, collagen, a colloid, hormones, preservatives, sweetener, superoxide dismutase (SOD), glutathione, and/or the like.

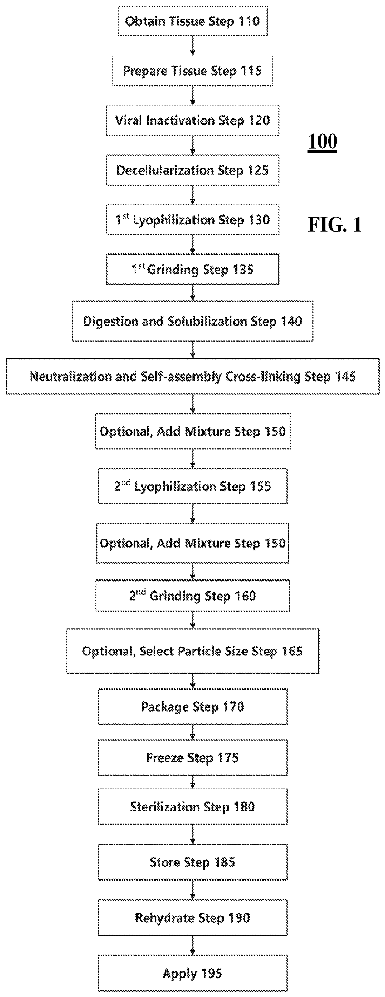

The rehydrated TH Powder with a reproducible gelation is sometimes referred to herein as a carrier matrix as its porous structure can carry the added components such as pharmaceutical drugs and therapeutics agents for sustained release. Various combinations of these components may be added during a Step 145 (Neutralization and Self-assembly Cross-linking, See FIG. 1), or be added during a Step 155 (Lyophilization) and then during Step 160 be ground together to produce a mixture dry powder. In various embodiments, the TH Powder has a shelf life of 3, 4 or 5 years or greater. In various embodiments, the TH Powder includes human or animal placental tissue.

Specific hormones that may be included in the TH Powder include growth hormones, interferon, adrenocorticotropic hormone (ACTH), cortisol, estrogen, kisspeptin, leptin, melanocytestimulating hormone (MSH), melatonin, norepinephrine, oxytocin, Peptide YY, progesterone, prolactin, prostaglandins, relaxin, serotonin, somatostatin, thyroid hormones, vitamin D, and/or the like.

Optionally, the TH Powder is configured to increase solubility, flowability and injectability following mixing with polyethylene glycol, or irradiated by Gamma or Electron Beam with a dose of at least 10, 15 or 20 kGy or higher.

Optionally, the TH Powder is configured to increase weight and/or density following mixing with additives, for example, sodium hyaluronate, carbomer, polysaccharides, hydroxyethylcellulose, polysaccharides, and/or chitosan.

Optionally, the TH Powder is configured to increase viscosity, adhesiveness, and shorten the polymerization and solidification time, increase weight and density, and prolong biodegradation rate, following mixing with additives, for example, hyaluronic acid, polyethylene glycol, sodium carboxymethyl cellulose, hydroxyethylcellulose, polysaccharides and chitosan, or being exposed to air, being applied to a patient, or mixing with an activation agent.

Optionally, a novel single-patient multi-use TH Powder includes preservatives. Existing commercial collagen or acellular tissue powder wound care products in the market are only for single use and applied as a powder, paste or a flowable matrix suspension (for example, Integra^{®} Flowable Matrix, ACell MicroMatrix^{™}). Preservatives such as potassium sorbate can be included in the TH Powder as a single-patient/multi-use spray or enema irrigation for skin, oral, vaginal, urethral, anorectal and GI track wound care. Ophthalmic preservatives such as Polyquaritum-1 can be included to the TH Powder for single-patient/multi-use ocular surface wound care, for example, managing corneal ulcerations, corneal abrasions, or easing the symptoms of dry eye.

In various embodiments, and as illustrated by the examples herein, the components of the powder, hydrogel, paste, liquid or solution may be added in any orders, before, during or after steps of lyophilization, cryogrinding, freezing, sterilization or rehydration.

FIG. 1 illustrates Methods 100 of manufacturing TH Powder, according to various embodiments of the invention. The illustrated methods are optionally adapted to generation of therapeutic pastes or solutions, by varying an amount of rehydrating liquid and or by varying production of the TH Powder using any of the methods discussed herein. The methods include producing the powder ground to achieve desirable characteristics, optionally sterilizing the powder, and rehydrating the powders for a variety of therapeutic uses, some of which are described further elsewhere herein.

The **Methods 100** illustrated in FIG. 1 are optionally performed in alternative orders.

In an Obtain Tissue Step 110, tissue having desirable properties is obtained. The tissue is porcine placental tissue. Alternatively, but not according to the invention claimed, the tissue includes human tissue such as placental tissue from humans, swine, fowl, sheep, suidae, equine, bovine, ovine, murine, molluscs, amphibians, rabbit or other mammals or fish, non-human animal embryonic tissue, and/or other suitable sources. Cold chain shipping delivery in ^{~}-10 Degrees Celsius is desired. For example, the porcine placentas are preferably collected and treated as follows within 1, 2 or 3 hours after removal from a source organism (e.g., after the birth of the last piglet, or after birth of a child, or after removal from a living organism); remove debris; rinse the porcine placentas in clean water; and immerse the porcine placentas in an antibacterial, antiviral and/or antibiotic solution (e.g., 0.01^{~}1% peracetic acid) optionally for at least 0.5, 1, 2 or 3 hours prior to freezing. Prompt freezing is helpful in reducing the initial bacterial bioburden. The placentas (e.g., placenta, amniotic membranes, amniotic sac or umbilical cord) can also be obtained from rabbits, sheep, cattle or other mammals. Further, the tissue optionally includes one or more of amniotic sac tissue, amniotic fluid, ocular tissue, lymph node tissue, neural tissue, umbilical cord tissue, and/or the like. For example, in various embodiments, extracellular matrix is derived from varied human and/or animal tissues or fish, for example and without limitation, placenta, amnion, chorion, umbilical cord, embryotic tissue, ocular tissue, lymph node tissue, neural tissue, skin, dermis, urinary bladder, small intestine, mesothelium, pericardium, heart valve, fascia lata, liver, lung, heart, adipose, skeletal, blood vessel, nerve conduits, cartilage, breast, colon, or other tissues and organs. Tissue includes skin, dermis, urinary bladder, small intestine, mesothelium, pericardium, heart valve, fascia lata, liver, lung, heart, adipose, skeletal, blood vessel, nerve conduits, cartilage, cornea, breast, colon, placenta, amnion, stem cells, and/or other tissues and organs.

In an optional Prepare Tissue Step 115, optionally after thawing or multiple freeze-thaw cycles (e.g., 2, 3, 4 or more freeze-thaw cycles), the tissue is washed in the flow of DI water, weighed and then inspected. For example, porcine placenta is obtained in Step 110, inspection standards of porcine placenta include fresh fishy smell, bright red color and no obvious odor. Optionally, the unwanted matters such as umbilical cords, large blood vessels and the amniotic membranes are physically removed. For example, in the flow of DI water, the tissue may be rubbed repeatedly with a mesh for 5 min, carefully remove the unwanted matters.

In a Viral Inactivation Step 120, the tissue is processed by one or any combination of the following: (1) a 0.15%^{~}0.3% peracetic acid and 2%^{~}3.5% ethanol working solution for a minimum of 2 hours exposure time; (2) irradiation such as Electron Beam or Gamma with a dose range of 1^{~}50 kGy, or Ethylene Oxide with a temperature below 50 Degrees Celsius for a minimum of 10 hours with an Ethylene Oxide concentration range of 450 ^{~} 800 mg/L in a relative humidity 50^{~}80% RH; (3) a heat treatment at a minimum of 70 Degrees Celsius for a minimum of 30 min exposure time; (4) a heat treatment at a minimum of 60 Degrees Celsius for a minimum of 2 hours exposure time; (5) a pH of 2.6 or less for a minimum of 18 hours exposure time; (6) a pH of 13 or greater for a minimum of 18 hours exposure time; (7) immersion in a minimum of 0.2% glutaraldehyde; (8) fixed in a minimum of 10% formalin; (9) treatment with proteinase K, followed by a heat treatment at 95 Degrees Celsius for 15 min, followed by RNase.

In a Decellularization Step 125, decellularization can be accomplished by many methods, including:

physical methods such as: (1) multiple freeze-thaw cycles result in ice crystal formation in the outer parts of cells, which causes the interior of the cells to expand, pushing against the plasma membrane until the cell bursts; (2) massaging the tissue on mesh results in high-hydrostatic pressures disrupting cells inside the tissue; and (3) non-thermal irreversible electroporation disrupts cell membranes while sparing all other tissue components.

chemical methods such as: (1) Alkaline and acid treatments are used to solubilize the cytoplasmic component of the cells as well as remove nucleic acids such as RNA and DNA; (2) hypotonic and hypertonic solutions promote cell lysis by osmotic shock, and also results in the disruption of DNA-Protein interactions; (3) non-ionic detergents, for example, t-octylphenoxypolyethoxyethanol (Triton X-100), disrupt DNA-protein interactions, disrupt lipid-lipid and lipid-protein interactions and, to a lesser degree, disrupt protein-protein interaction; (4) ionic detergents, for example, sodium dodecyl sulfate, sodium deoxycholate, solubilizes cell and nucleic membranes, and tend to denature proteins; (5) zwitterionic detergents, for example, 3-[(3-Cholamidopropyl) dimethylammonio]-1-propanesulfonate (CHAPS), sulfobetaine-10 and -16 (SB-10, SB-16), have characteristics of both ionic and non-ionic types; (6) alcohols cause cell lysis by dehydration, solubilize and remove lipids; (7) acetone lyses cells by dehydration, solubilizes and removes lipids; and (8) tributyl phosphate (TBP) forms stable complexes with metals, and disrupt protein-protein interactions.

biologic methods such as: (1) enzymes, for example, nucleases which catalyze the hydrolysis of ribonucleotide and deoxyribonucleotide chains, or trypsin which cleaves peptide bonds on the C-side of Arg and Lys, or dispase which cleaves specific peptides, mainly fibronectin and collagen IV; and (2) non-enzymatic agents such as chelating agents, for example, ethylenediaminetetraacetic acid (EDTA) or egtazic acid (EGTA) which binds metallic ions, therefor disrupts cell adhesion to extracellular matrix, or protease inhibitors, for example, phenylmethylsulfonylfluoride, aprotinin or leupeptin, inhibits many proteases needed to maintain the native extracellular matrix ultrastructure.

In a 1^{st} Lyophilization Step 130, the decellularized tissue is lyophilized through an industrial size lyophilizer, which can be programmed to multiple temperature steps that the temperatures are reduced gradually, rather than laboratory size or small scale lyophilizers that only have one programmed temperature. The multiple-temperature computed and programmed lyophilization reduces chamber pressures gradually, thus avoids product collapse and degradation, and also avoids the dried tissue being re-wetted by the condensed water in the air, which is a common problem in the laboratory size or small scale lyophilizers. As used herein, a multi-temperature programmed lyophilization device is one in which a temperature program, including more than one temperature, can be programmed. For example, lyophilization occurs as the temperature of the tissue is controlled over a pre-programmed range of temperatures (e.g., more than one temperature).

In a 1^{st} Grinding Step 135, the tissue is ground at low temperature of less than 0 degrees C., preferably of less than -10 degrees C, less than -20 degrees C, or dry ice temperatures, to produce a micronized powder. Several tissue grinding systems and methods have been developed. In various embodiments, these cryogrinding systems and methods result in a dry powder having 50% or more particle diameter sizes less than 200 µm, which lead to a fast and complete digestion in Step 140 (below) that doesn't require a removal of insoluble matter. It is a greater improvement compared to previous publications, for example, U.S. Application Pat. Pub US20080181967A1 Liu et al. which lacks grinding and typically requires a removal of insoluble matter. 1^{st} Grinding Step 135 may result in particles less than or greater than 200 µm.

In a Digestion and Solubilization Step 140, the ground tissue is digested and solubilized. Multiple digestion methods can be used in this step. For example, solubilization protocols are referred to as "Voytik-Harbin, Freytes and Uriel" disclosed in "Extracellular Matrix Hydrogels from Decellularized Tissue: Structure and Function" Saldin et al. In another example, digestion and solubilization is accomplished using an alkaline solution disclosed in US Pat. No. 8,802,436 Kentner et al. Optionally, digestion and solubilization at a neutral pH using collagenases which function extracellularly and cleave polypeptide chains in the collagen triple helix at specific loci resulting in solubilization. 1^{st} Grinding Step 135 aids in the digestion and/or solubilization of Digestion and Solubilization Step 140 as it, for example, makes more surface area of the tissue available for reaction.

In a Neutralization and Self-assembly Cross-linking Step 145, the digested solubilized tissue (now a hydrogel) is then neutralized, for example, neutralized by 0.1M sodium hydroxide to form a hydrogel having a pH that is more than or equal to 6.5 pH and is less than 7.2. For digestion and solubilization at a neutral pH in Step 140, then Step 145 is not needed. Following Digestion and Solubilization Step 140 or Neutralization and Self-assembly Cross-linking Step 145, the digested tissue typically forms an (intermediate) hydrogel.

In some embodiments, additional components are added to the neutral hydrogels in Add Mixtures Step 150. The additional components can include, for example, any of the pharmaceutical agents, therapeutic agents, preservatives, and/or other materials discussed herein. Addition may occur during mechanical mixing.

In one example, the hydrogel derived from Step 145 may be combined with growth hormones, optionally, stem cells, exosomes, a collagen thickener, antibiotics, or a light activated cross-linking agent. In another example, the TH Powder can directly mix with the liquid growth hormones, antibiotics, , anti-inflammatory drugs, chemotherapy drugs, anti-VEGF drugs bevacizumab and ranibizumab, growth factor drugs, anti-metabolites, and/or the like for rehydration upon the point of use. The hormone may be selected to promote growth, to reduce inflammation, to minimize neovascularization, to stimulate stem cell growth, to promote nerve or blood vessel growth. Polymerization and solidification (reproducible gelation) of the TH Powder may be initiated by rehydration, meeting the temperature ^{~}25 Degrees Celsius or higher, light, exposure to air, or mixing with an activation agent.

In a 2^{nd} Lyophilization Step 155, the neutral hydrogel is lyophilized, which results a sponge-like structure. Add Mixtures Step 150 is optionally repeated or first performed following 2^{nd} Lyophilization Step 155. 2^{nd} Lyophilization Step 155 occurs after 1^{st} Grinding Step 135.

In a 2^{nd} Grinding Step 160, the sponge is micronized through cryogrinding to produce a dry powder, including a final particle size having 50% or more particle diameter sizes less than 50, 100, 150 or 200 µm. In some examples not covered by the claims, 2^{nd} Lyophilization Step 155 and/or 2^{nd} Grinding Step 160 are optional. One result of 2^{nd} Grinding Step 160 is a further reduction in particle size (relative to that produced in 1^{st} Grinding Step 135), which is desirable for several of the clinical applications discussed herein. Grinding both before and after Digestion and Solubilization Step 140 first provides for better digestion and second allows for grinding to smaller sizes once digestion has occurred.

Optionally, 2^{nd} Grinding Step 160 includes selection of a target particle size (and/or distribution) of the powder based on grinding conditions., Further, 2^{nd} Grinding Step 160 is optionally followed by a Select Particle Size Step 165 in which a subset of particles of the powder produced in 2^{nd} Grinding Step 160 are selected for their particle size, e.g., using a mesh or any other particle size sorting device, e.g., electrostatic/electrodynamic sorter or settling sorter. Particle size and distribution are optionally selected specifically for various clinical applications. For example, in aesthetics mesotherapy which requires injections through 27-30 gauge needles, then particle sizes with diameters less than 40 µm or between 30-40 µm are selected for use in further processing in the methods illustrated by FIG. 1. (Unselected particles optionally being discarded.) For example, particle size and/or distribution may be selected to achieve a desired solubility, flowability, and/or injectability, and/or the desired time of polymerization and solidification when meeting the average body temperature. With smaller particles, the solubility, flowability and injectability of the rehydrated TH Powder is increased, and the time of polymerization and solidification when meeting the average body temperature is shortened. Solubility has been found to be controllable by selecting both particle size and size distribution, smaller particles being easier to solvate. For example, the intravitreal injection to treat retinopathy and wet, AMD, or macular holes optionally carrying bevacizumab or ranibizumab, requires the TH Powder to dissolve evenly and quickly, on-site during the surgery or at least within 4 hours prior to the surgery due to the critical standards of keeping the product sterile. However, the generation of smaller size particles is limited by the goal of preserving biological properties of the material to be ground. Too much grinding can substantially reduce biological properties of the material. Too much grinding and/or poor grinding conditions can also reduce biological properties of the material. Thus, particle size may be selected for specific applications, further examples of which are discussed elsewhere herein. Steps 130/135 and 155/160 represent at least two lyophilization/grinding cycles. Additional lyophilization/grinding cycles are possible in alternative embodiments.

In an optional Package Step 170, the powder is packaged in a sealed glass vial, a plastic bottle, a plastic spray bottle, a plastic eye dropper bottle, a vaginal or anorectal applicator, a syringe, or the like. Optionally, the sealed vials or bottles can be under vacuum or under an inert gas. Optionally, the powder is microencapsulated after grinding. Microencapsulation is alternative method of packaging of the powder.

In an optional Freeze Step 175, the (optionally packaged) powder (TH Powder) is frozen to - 10 Degrees Celsius or lower temperatures, optionally for a minimum 12 hours, or packaged with dry ice. In alternative embodiments the TH Powder is frozen to less than 0, -5, -15, -20 or -60 degrees Celsius. In alternative embodiments, the TH Powder is frozen for at least 1, 4, 6, 8, 10, 15 or 24 hours. The time of freezing may depend on packaging structure and/or heat transport through the powder. In some embodiments Freeze Step 175 includes freezing the powder to approximately -80 C with a preferred time of 36 hours, and/or packaged with dry ice.

In a Sterilization Step 180, the powder is sterilized. This is done by using irradiation such as EB or Gamma. In various embodiments, such Irradiation is performed with a dose range of at least 1, 5, 10, 15, 25 or 50 kGy, or any range therebetween. The powder is optionally moved relative to the irradiation source during irradiation.

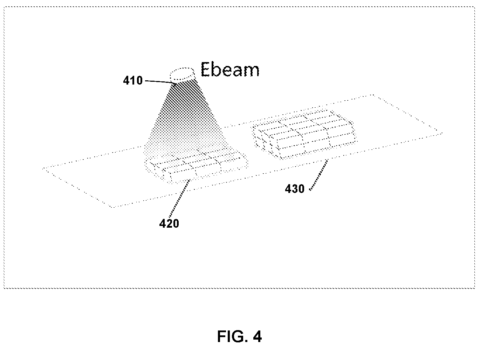

In some embodiments, for maintaining the biological properties of the TH Powder, a irradiation dose range of 15-25 kGy, a novel carton packaging system (described elsewhere herein), freezing for minimum 12 hours and optionally packaging with dry ice is used for Electronic Beam (EB) irradiation (See, for example, FIG. 4). Under some conditions, irradiation doses above 25 or 15 kGy do not result in a reproducible gelation of the powder on rehydration. Under some conditions, irradiation doses below 15 kGy result in a reproducible gelation. ETO sterilization, with a maximum temperature of 50, 37 or 25 Degrees Celsius or below, for a minimum of 10 hours with a concentration range of 450^{~}800mg/L in a relative humidity 50^{~}80% RH, results in a reproducible gelation of the rehydrated TH Powder while maintaining terminal sterility and minimizing the residue of ETO. In previous embodiments, the selection of the sterilization methods were discussed to fit various clinical application demands. Optionally, the powder is kept below room temperature or below (or near) the freezing temperature of Freeze Step 175 during Sterilization Step 180.

The TH Powder, of various embodiments of the invention, is highly dissolvable, and can be rehydrated (in an optional Rehydrate Step 190) in a glass vial, a plastic spray bottle, a plastic container bottle, a plastic eye dropper bottle, a vaginal or anal applicator; to produce a paste, a liquid, a spray, a drop, an injection or an enema irrigation upon addition of a solution such as purified water, normal saline, phosphate-buffered saline (PBS), sterile irrigating solution, balance salt solution (BSS), other bio-compatible salt solutions that may contain sodium chloride and/or potassium chloride, and other liquid pharmaceutical drugs or therapeutic agents. The rehydrated TH Powder may be applied for therapeutic uses as described elsewhere herein. For example, the powder may be stored in a dried form and later mixed with a liquid to form a solution, paste or hydrogel. The 2^{nd} Grinding Step 160 is optionally repeated multiple times to achieve desired powder particle size and/or distribution. Optionally, the rehydrated TH Powder is added to a device implantable in a human body which optionally includes a sensor, a wire, a joint, a bolt or screw, a syringe, a bio-scaffold, a canula, a catheter, a pacemaker or a stent. The rehydrated TH Powder is optionally configured to polymerize and solidify and to form a protective film or a gel on the device and/or patient. For example, rehydrated TH Powder may be applied to a steel needle or cannula of an insulin infusion device. For example, rehydrated TH Powder may be applied to a stent configured to be disposed within an Artery, Vein, a urethra stent, wound drain, a stoma stent, a bronchial stent, a dermal stent, and/or the like. The rehydrated TH Powder is optionally used in conjunction with a vacuum assisted wound closure system.

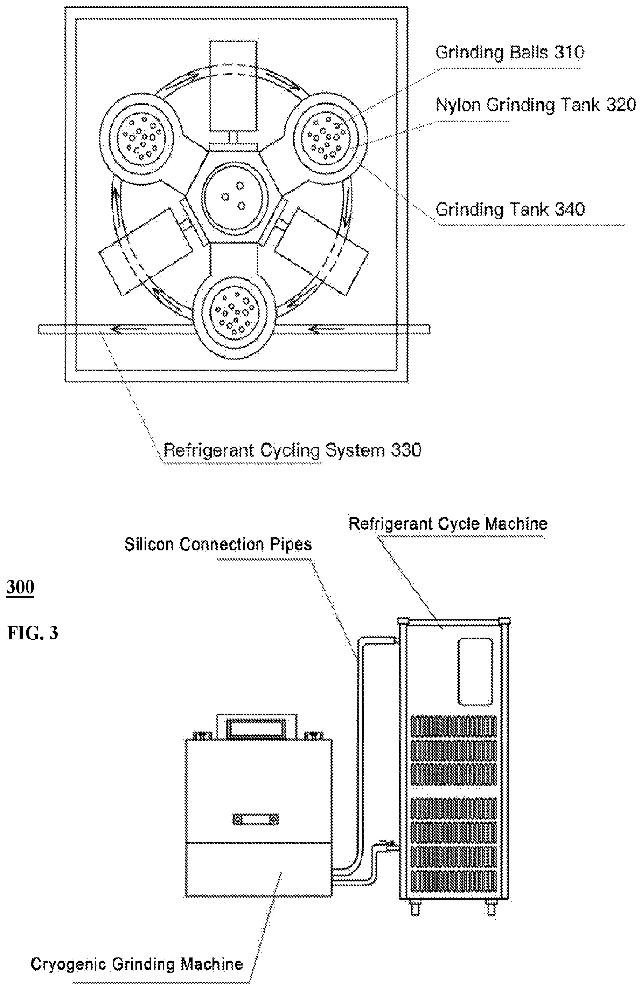

Grinding Steps 135 and 160 are optionally performed at low temperatures (cryogenic grinding) and the materials being ground can include freeze-dried components. For example, as discussed further elsewhere herein, the grinding may be performed in a temperature-controlled grinder configured to maintain the ground materials in various temperature ranges (optionally below room temperature) during the grinding process. The temperature is optionally selected to preserve biological and/or therapeutic properties of the components being ground. Further details of the Grinding Step 135 and 160 that are performed at low temperatures (cryogenic grinding) are discussed elsewhere herein, for example with respect to FIG. 3.

In some embodiments, Sterilization Step 180 is performed through irradiation including electron beam or gamma radiation. Alternatively, the irradiation includes alpha particles, high energy electrons, high energy ions, neutrons, protons, , x-rays or ultraviolet light. Freeze Step 175 optionally helps maintain the biological properties of the tissue powder while the irradiation produces heat that may cause damages to the biological properties, i.e., Freeze Step 175 may continue during Sterilization Step 180 to maintain a freezing temperature of Freeze Step 175.

In some embodiments, a typical EB dose range may be between 15 ^{~} 30 kGy to reach terminal sterility (SAL 10-6). However, an EB dose range between 15 ^{~} 25 kGy can be sufficient to reach to the terminal sterility in the novel carton box packaging system included in some embodiments of the invention. Optionally, the glass vials containing the TH Powder may be under vacuum or under an inert gas during sterilization.

In an optional Store Step 185, the TH Powder is stored for future use. A shelf life of the TH Powder may be extended by storage at a controlled room temperature, storage below 10, 5 or 0 Degrees Celsius storage, and/or addition of preservatives, such as antioxidants. In some embodiments, storage takes place in a sealed vial and/or a delivery device (See, for example, the delivery devices discussed in Apply Step 195).

In an optional Rehydrate Step 190, the TH Powder is rehydrated with a solution, proximate to a time of intended use. The amount of liquid used to rehydrate the TH Powder can be selected to determine whether the resulting product is a paste, a liquid, a spray, a hydrogel or a carrier matrix carrying pharmaceutical drugs and therapeutic agents. The amount of liquid can also be used to control viscosity and adhesiveness of the rehydrated TH Powder. In various embodiments, liquids used to rehydrate the TH Powder include water, saline, normal saline, hypertonic saline, buffered saline, phosphate-buffered saline (PBS), sterile irrigating solution, balance salt solution (BSS), buffered liquids, edible oils, juices, dairy products, other bio-compatible salt solutions that may contain sodium chloride and/or potassium chloride, and other liquid pharmaceutical drugs, and/or the like. The fluid used is optionally selected based on the intended use. For example, the salt concentration and the pH of the rehydrated TH Powder may be adjusted to match a normal pH and osmotic pressure of a part of the body in which treatment is intended to occur (pH and osmotic pressure being different in the eye relative to the heart or vagina, etc.). Rehydrate Step 190 optionally includes selecting a volume of liquid to achieve a desired viscosity of the rehydrated powder.

In some embodiments, the Rehydrate Step 190 is performed immediately prior to use and the rehydration is nearly instant after several shakes of the containers. Also, as noted elsewhere herein, the smaller particle size of the TH Powder results in much shortened polymerization and solidification time within 3 to 5 minutes at normal body temperature. This is in sharp contrast to the traditionally prepared liquid tissue derived hydrogels or lyophilized hydrogel sponges that polymerize and solidify around 20 to 30 minutes.

The Rehydrate Step 190 is optional if the TH Powder is used therapeutically in a dried powder form on the skin wet wound or in postoperative cavities. In such cases rehydration occurs when the TH Powder comes in contact with body fluids.

In an Apply Step 195, the powder, or a paste, a liquid, a spray, a hydrogel or a carrier matrix derived therefrom the TH Powder is used in a therapeutic, nutritional or cosmetic application. These applications may vary widely, some illustrative examples are discussed elsewhere herein.

In various embodiments of the Apply Step 195, the TH Powder is added to a mechanical delivery device, optionally after rehydration, to create a therapeutic delivery system, according to various embodiments of the invention. In a simple example, the TH Powder is stored in a sterilized and sealed glass vial. In various embodiments, the TH Powder is included in a cosmetic, a dermal cream, a sunscreen, a wound dressing, a bandage (for example, a hydrocolloid gel bandage or a Band-Aid^{™}), an eye drop, an anti-inflammatory cream, an antipruritic cream, a wound closure strip, surgical sponge, tampon, suppository (anal or vaginal), an injection device, an intrauterine device, a stent, a catheter, an transdermal cannula, a nasal or oral cannula, a transdermal patch, a device configured to be implanted in a living person or animal, and/or the like. As noted elsewhere herein, the TH Powder is optionally rehydrated in a glass vial, a plastic spray bottle, a plastic container bottle, a plastic eye dropper bottle, a vaginal or anal applicator to form a spray, an eye drop, an enema irrigation, an injectable and flowable hydrogel, or an injectable and flowable carrier matrix carrying various pharmaceutical drugs and therapeutic agents. In various embodiments, the TH Powder may be delivered using an injection device, cosmetic dispenser, a micro-needle or array thereof, a spray device, a nebulizer, a catheter, a stent, added to a surgical staple or suture material (absorbable or non-absorbable), a drinkable solution (optionally including flavoring), and/or the like. In any of these mechanical delivery devices or compositions, the TH Powder may be included dry or rehydrated to a paste, a hydrogel, a spray, a gel, and/or a liquid solution, as appropriate for the particular system. In some embodiments, an end user can select between a hydrogel or a paste or a liquid solution by selecting a rehydration volume and concentration. For any concentrations lower than 20mg/mL, it cannot result in a reproducible gelation. However, the low concentration of the rehydrated TH Powder, such as 5mg/mL, 8g/mL or 10mg/mL, can result in a fast drying and then become a thin layer of protective film that temporarily adheres to skin surface or mucosal membranes, which can provide a moist environment for wound healing and a temporary relief from irritations.

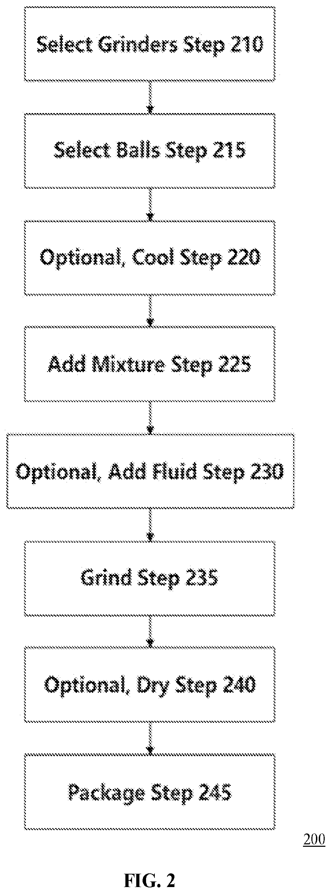

FIG. 2 illustrates novel grinding methods 200, according to various embodiments of the invention. These methods are optionally performed as part of the Grind Steps 135 and/or 160 illustrated in FIG. 1. In these methods a mixture of tissue and any of the other optional components discussed herein are ground to a powder of desired characteristics. For example, porcine placental tissue may be ground to a fine powder, which when rehydrated forms a therapeutic solution, paste, hydrogel and the like. In various embodiments, the powder is ground to a size distribution as discussed elsewhere herein (See, for example, Table 2). Grinding can be accomplished in either a patch process or a continuous process. Grinding is performed at a reduced temperature. A cooling system is used to maintain the ground mixture at temperature below -20 Degrees Celsius during grinding. See the examples herein for temperature ranges used in various embodiments. The tissue and/or other materials ground are optionally freeze-dried prior to grinding. Grinding may be performed using dry grinding (Table 1-2) or wet grinding (Table 3) methods. In wet grinding methods, a grinding liquid is added to the mixture to be ground. The steps illustrated in FIG. 3 are optionally performed in alternative orders. For example, selection of grinding implements may be performed before adding materials to be ground.

In various embodiments of the invention, the Methods 200 illustrated in FIG. 2 are used for batch grinding of tissue. In batch grinding, one batch of material is ground at a time. Typically, batch grinding is performed in a large container, alternatively referred to herein as a tank, vessel, or vat. This container may be open, closed or sealed during grinding. In a Select Grinders Step 210, one or more grinding implements are selected for use in the continuous grinding system. These grinding implements may include any combination of the grinding implements discussed elsewhere herein.

In various embodiments of the invention, the Methods 200 illustrated in FIG. 2 are used for continuous grinding in a continuous grinding system. In these embodiments, material is continuously provided to an input of the grinding system and a ground powder is continuously expelled at an output of the grinding system. Ground powder may exit the output at the same time that additional material is provided at the input.

In an optional Select Balls Step 215, selecting one or more grinding balls are selected and adding to the grinding tank. As noted elsewhere herein, the grinding balls may be selected based on a desired particle size and distribution in the powder that results from grinding.

In an optional Cool Step 220, the grinding tank is cooled. This cryogenic grinding at low temperatures may be accomplished, for example, by circulating a refrigerant within a double wall of the grinding tank. The refrigerant may be any of those known in the art of refrigerant systems. In various embodiments, the interior of the grinding tank is maintained during grinding at temperatures less than 10, 0, -15, -25, -30, -40 or -50 Degrees Celsius, or any range therebetween. This cooling may be performed, for example, by passing a refrigerant through a cooling jacket of the continuous grinding system or by adding a cooling liquid to the material being ground.

In an Add Mixture Step 225, the mixture to be ground that may include various materials discussed elsewhere herein is added to a grinding tank. The materials (e.g., tissue) to be ground are added to the continuous grinding system at an input. Optionally, the output of a freeze-drying system is fed directly to this input. In some embodiments the continuous grinding system includes multiple inputs at which different components to be ground can be added. For example, freeze-dried decellularized tissue may be added at a first input port and a preservative, an antibiotic and stem cells may be provided at one or more different inputs to the continuous grinding system. As such, different components of the mixture may be subject to different types and amounts of grinding.

In an optional Add Fluid Step 230, a wet grinding process refers to a grinding fluid is added to the grinding tank. This grinding fluid comes in contact with the material being ground. The grinding fluid may serve as a lubricant during the grinding process, resulting in a smaller particle sizes and narrower particle size distribution, and shortening grinding time needed to reach a desired particle size (Table 3). The grinding fluid may also serve to maintain thermal transport and equilibrium within the material being ground. The grinding fluid is optionally also a cooling fluid. For example, the cooling fluid may be pre-cooled to a desired grinding temperature, or may include an ice bath configured to maintain the desired temperature. The grinding fluid is preferably a liquid at the desired grinding temperature when combined with the material to be ground. The grinding fluid optionally includes saline, normal saline, a salt solution, one or more alcohols or any of the suitable cooling fluids or refrigerants discussed herein. The grinding fluid is optionally added at a different input to the grinding system relative to the tissue to be ground.

In a Grind Step 235, stirring rotors are used to move the grinding balls within the grinding tank to grind the material into a powder of the desired particle size and size distribution. In various embodiments, grinding speeds between 500-3000 rpm, 1000-3000 rpm, 1800-2000 rpm or 1500-2500 rpm are used. Other speeds may also be used depending on the size and other characteristics of the grinders. Grinding may continue until the desired particle size is achieved, for example, the grinding may take between 5-30 min, or 10-15 min pending on various equipment selected in Step 210 and 215. The resulting particle size is directly related to the grinding time. The longer grinding takes, the smaller the particle sizes can be achieved.

In an optional Dry Step 240, the ground material through wet grinding is dried/lyophilized to remove (for example, evaporate or sublimate) the grinding fluid or liquids found in the original material to be ground. Dry Step 240 may be performed within part of the grinding system or within a separate equipment such as lyophilizer. Drying is optionally performed under negative pressure, e.g., vacuum. For example, 1000mL saline is added to a 1000mL tissue derived hydrogel (20mg/mL) in the wet grinding process in in Step 235, and the ground mixture is lyophilized thus the 1000mL added saline is evaporated and the ground mixture remains 20mg/mL concentration.

In an optional Package Step 245, the ground material (dry powder, dry sponge or a liquid) is added to a suitable container. Package Step 245 is optionally an embodiment of Package Step 170.

FIG. 3 illustrates an exemplary batch oscillating grinding system 300 including Grinding Balls 310, a Nylon Grinding Tank 320, a Refrigerant Cycling System 330 and a Temperature Controllable Grinding Tank 340 according to various embodiments of the invention. The refrigerant is optionally supplied using a refrigerant compressor, silicon transfer tubing and suitable temperature control electronics.

Oscillating grinding system 300 (which is optionally used in the Grinding Steps 135 and 160) of various embodiments of the invention involving the fast movements of the grinding balls is highly efficient and effective, produces small particle sizes and grinds materials in all forms, for example, wet liquids, dry materials, high fibric materials such as cotton, meat or vegetables. It is a great improvement compared to previously used planetary grinding systems in which the grinding tank rotates on an orbit around the center. In batch grinding systems, a quantity of material is ground together, for the entire quantity the grinding starts at the same time and ends at the same time. The novel oscillating grinding systems can be divided into "batch" and "continuous" systems. Batch grinding may occur in a container, such as a bowl-shaped vessel. In addition to the material to be ground, grinding balls may be added to the container. These grinding balls are optionally moved using stirring rotors, for example, Ø10*30mm. In continuous grinding systems, material to be ground is provided at an input and ground product exits an output. Examples of continuous grinding systems include screw grinders. Such grinders can include regions having different grinding implements. These implements can include, for example, feeder structures (for example, one, two or more screws) configured to drive material forward, region including burrs, regions including blades, regions include augers, regions including gears or other meshing parts, tapered regions, regions with rollers, regions including mincer augers, and/or regions including grinding balls. Different regions may rotate at different speeds. For example, a continuous grounder may include feeder regions separated by roller and/or grinding ball regions. While batch grinding systems are discussed herein for the purpose of example, the teachings and examples provided are readily applied to continuous grinding systems. For example, the cooling systems described herein may be used for batch and continuous grinding systems.

Characteristics of the grinding process that can be controlled to optimize the resulting product include grinding speed, grinding ball sizes, grinding region structure and dimensions, grinding taper, vessel temperature, grinding fluid formula and temperature, grinding time, and/or the like. In various embodiments, these characteristics are chosen to achieve a desired particle size and distribution while also preserving the biological properties of compounds within the resulting powder. Specifically, a powder size small enough to have an excellent solubility, flowability and injectability and a short polymerization and solidification time is achieved while maintaining the grinding process at a temperature at which biological properties are maintained.

In an illustrative embodiment, a temperature controlled grinding tank has a double wall and includes connections configured for flow of a refrigerant between the walls. The inner and outer walls may include different materials, for example, nylon and stainless-steel, respectively. Grinding balls of one or more sizes are placed in the tank during grinding. Typically, the grinding ball(s) tank and/or other grinding parts comprise a hard material such as zirconia, nickel, titanium, carbide and/or stainless-steel (for example, 316L SS). Several, 1, 2, 3, 4 or more different sizes of grinding balls may be used in the same operation. For example, 1, 2, 3, 5, 8 and 10 millimeter (diameter) grinding balls may be used together, in various combinations, in a cryogenic grinding process. The number and sizes of the grinding balls may be selected to generate a desired medium, average particle sizes and desired size distribution. In some embodiments, a refrigerant such as an ethanol solution, for example, 99.7% EtOH, is used as a refrigerant to keep the grinding tank at a temperature between -30 and -90 Degrees Celsius; 100% EtOH, is used as a refrigerant to keep the grinding tank at a temperature between -90 and -114 Degrees Celsius. Alternative refrigerants may include, for example, dry ice, ethylene glycol, acetone, water, ethanol, o-Xylene, m-toluidine, acetonitrile, pyridine and methanol. The flow of the refrigerant may be managed by temperature control electronics. Stirring rotors are used to move the grinding balls, for example at 1000-2000 rpm (rotations per min).

In some embodiments, the grinding balls have three different diameters of 10 mm, 5 mm and 2 mm, respectively, and the quantity of the grinding balls used is in the respective ratio 1:2:3. One, two, three or more sizes of grinding balls may be used. The grinding balls may have different densities, for example, smaller grinding balls having higher densities. In some embodiments, the size ratio was tested among the three different sizes 1:2:3. Of course, it can be 1:5:10, 1:3:6, 1:4:20, or other ratio combinations. Three size grinding balls can maximize the efficiency and effectiveness of the grinding process. Various embodiments include at least two or at least three sizes of grinding balls. The largest to smallest having ratios of at least 1:3, 1:4, 1:5 or 1:10 in diameter. Optionally, the diameters of the grinding balls and the ratios among the grinding balls are selected based on the desired powder particle size.

In some embodiments, the novel oscillating grinding systems includes adding long vertical handles to the grinding tanks (Nylon Grinding Tank 320) to avoid direct contact thus to reduce any risks in introducing contamination to the ground materials in the grinding tanks.

Various embodiments of the invention include the use of dry batch grinding to produce a powder with a particle size that can be controlled while maintaining desirable therapeutic properties of the native tissue at levels not previously demonstrated by other inventions.

Dry grinding test results (Table 1 and Table 2).

To determine the effect of the ratio of four sizes of grinding balls on powder particle size in the dry grinding process, totaling 9 different ratios of four sizes of grinding balls were tested as shown in Tables 1 and 2.

**Table 1**

| No. | Grinding ball | | | | Sample^{[3]} (g) | Time (min) | Speed (rpm) | Method |
|---|---|---|---|---|---|---|---|---|
| | 8 mm^{[1]} (number) | 5mm (number) | 3 mm (number) | 1 mm (g)^{[2]} | | | | |
| 1 | 3 | 10 | 15 | 20 | 3 | 15 | 2000 | Dry grinding |
| 2 | 5 | 10 | 15 | 20 | 3 | 15 | 2000 | Dry grinding |
| 3 | 7 | 10 | 15 | 20 | 3 | 15 | 2000 | Dry grinding |
| 4 | 5 | 5 | 15 | 20 | 3 | 15 | 2000 | Dry grinding |
| 5 | 5 | 15 | 15 | 20 | 3 | 15 | 2000 | Dry grinding |
| 6 | 5 | 10 | 10 | 20 | 3 | 15 | 2000 | Dry grinding |
| 7 | 5 | 10 | 25 | 20 | 3 | 15 | 2000 | Dry grinding |
| 8 | 5 | 10 | 15 | 10 | 3 | 15 | 2000 | Dry grinding |
| 9 | 5 | 10 | 15 | 30 | 3 | 15 | 2000 | Dry grinding |

| | | | | | | | | |
|---|---|---|---|---|---|---|---|---|
| ¹ mm represents the grinding ball diameters in millimeter. ²1 mm-grinding balls were too small to be counted correctly within a short time. As a result, we used the weight to indicate the content of 1 mm-grinding balls. ³Samples referred to the weight of the dry tissue materials to be ground. | | | | | | | | |

After the novel cryogenic grinding process, the particle sizes of the ground tissue materials were assessed by Mastersizer 3000 laser particle size analyzer (Malvern Instruments Ltd., Malvern, UK) as being illustrated in Table 2.

**Table 2**

| **No.** | **Size** | | |
|---|---|---|---|
| | D₉₀ (µm) ^{[3]} | D₅₀ (µm) ^{[3]} | D₁₀ (µm) ^{[3]} |
| **1** | 443.462 | 162.881 | 44.340 |
| **2** | 345.670 | 151.941 | 43.849 |
| **3** | 289.838 | 130.724 | 36.016 |
| **4** | 520.735 | 188.415 | 54.570 |
| **5** | 323.845 | 162.996 | 40.682 |
| **6** | 393.342 | 155.825 | 44.582 |
| **7** | 262.125 | 129.571 | 36.295 |
| **8** | 424.315 | 187.628 | 52.948 |
| **9** | 341.194 | 156.604 | 40.697 |

| | | | |
|---|---|---|---|
| ³D₉₀: the maximum particle diameter below which 90% of the sample volume exists. D₅₀: the maximum particle diameter below which 50% of the sample volume exists. D₁₀: the maximum particle diameter below which 10% of the sample volume exists. | | | |

The test results in Table 2 showed: (a) under the same conditions, the more grinding balls, the smaller the powder particle sizes were achieved; and (b) the 8 mm- and 3 mm-grinding balls were more important than the other sizes to produce the small powder particle size.

Various embodiments of the invention include the use of wet batch grinding to produce a therapeutic hydrogel, a liquid or optionally a dried sponge. The test result of wet grinding (Table 3) showed a particle size that can be controlled while maintaining desirable therapeutic properties of the native tissue at levels not previously demonstrated by other inventions.

Wet grinding test results (Table 3).

In general, wet grinding is more efficient, and resulted in smaller and more uniform particle sizes of powder, compared to dry grinding. Wet grinding tests were performed for 15 min, with a grinding speed 2000 rpm, and adding 100 grams of 1 mm-grinding balls into the grinding tank. The resulting particle sizes were shown in Table 3.

**Table 3**

| No. | Grinding ball | | | | Size | | | Time (min) | Method |
|---|---|---|---|---|---|---|---|---|---|
| | 8 mm (number) | 5mm (number) | 3mm (number) | 1 mm (g) | D₉₀ (µm) | D₅₀ (µm) | D₁₀ (µm) | | |
| 10 | 0 | 0 | 0 | 100 | 135.501 | 39.218 | 9.927 | 15 | Wet grinding |
| 11 | 0 | 0 | 0 | 100 | 234.886 | 93.707 | 23.255 | 15 | Wet grinding |

| | | | | | | | | | |
|---|---|---|---|---|---|---|---|---|---|
| Various embodiments include powers having D50 as listed above in tables 2 and 3, or any range therebetween. | | | | | | | | | |

The test results in Table 3 showed: (a) smaller particle sizes of the ground materials were achieved by using grinding balls with smaller diameters; (b) wet grinding was more efficient than dry grinding.

Maintaining biological properties of the ground mixture.

Achieving small particle sizes is meaningless if the biological properties of the ground mixture cannot be maintained. Traditional grinding (as seen in the prior art) produces a lot of heat, including localized heat, which leads to loss and destruction of biological properties through processes such as protein denaturation.

To test biological properties, the largest and smallest particle size powder ground by the dry grinding process (Group No.4 and No.7 in Table 2) and the liquid ground by the wet grinding (Group No.10 and No.11 in Table 3) were compared to the lyophilized tissue in the Lyophilization Step 130 (Group No.0). The characterization of collagen (Soluble Collagen Assay Sircol^{™}, S1000, Biocolor, UK), elastin (Elastin Assay-FastinTM, F2000, Biocolor, UK) and GaGs (Glycosaminoglycan Assay BlyscanTM, B1000, Biocolor, UK) were illustrated in Table 4 (*p < 0.05 compared with No.0).

| No. | Collagen | Elastin | GaGs |
|---|---|---|---|
| | µg/mg dry weight | | |
| 0 | 375±17.4 | 187±20.4 | 7.68±0.15 |
| 4 | 370±29.6 | 173±10.3 | 5.88±0.16* |
| 7 | 360±13.3 | 179±22.6 | 5.81±0.15* |
| 10 | 368±13.5 | 177±13.9 | 5.69±0.02* |
| 11 | 365±7.4 | 177±10.2 | 5.73±0.03* |

These results indicated that the Methods 100 illustrated in FIG. 1, the Methods 200 illustrated in FIG. 2 and the System 300 illustrated in FIG. 3 produced desirable powder sizes and powder derived liquid having a greatly improved characteristics of small particle size and biological properties compared to previous inventions. As discussed elsewhere herein, the small particle size expands the properties and application scenarios for the TH Powder (for example, quick polymerization and solidification time with 3-5 min, instant rehydration upon the point of use). Specifically, the results indicated that the grinding methods produced a desirable powder while protecting collagens, proteins, GaGs and elastins and other therapeutic components from being degraded or destroyed. The grinding performed with different ratios of four sizes of grinding balls had no significant effect on the collagen and elastin characterization. However, GaGs were decreased as shown after grinding but were still present. The test results illustrated a fine balance between optimizing particle sizes and maintaining biological properties through the methods illustrated in FIGs. 1, 2 and 3, as achieved by various embodiments of the invention.

In various embodiments of the invention, the TH Powder (retaining biological properties) has a D50 (µm) of less than 250, 200, 150, 125, 100, 75, 50 or 39.3, or any range therebetween. In various embodiments of the invention, the TH Powder (retaining biological properties) has a D10 (µm) of less than 100, 50, 40, 24 or 10, or any range therebetween. In various embodiments of the invention, the TH Powder (retaining biological properties) has a D90 (µm) of less than 650, 500, 450, 400, 350, 250, 200, 150, or 136, or any range therebetween. In these embodiments, smaller D90, D50 and D10 may be achieved using different grinding implements, multiple grinding cycles and/or extended grinding time. A lower limit on the sizes results from the reduction in biological properties that would result from further grinding.

FIG. 4 illustrates a novel carton packaging system of the TH Powder for Electronic Beam (EB) irradiation, according to various embodiments of the invention. The illustrated system is optionally used for other types of irradiation discussed herein. An Ebeam (Electronic Beam) 410 is directed at an array of TH Powder packaging 420 containing TH Powder. The packaging is optionally configured to hold the TH Powder in one or two thin layers (having a thickness that is fully penetrated by at least 50, 75 or 90% of the Ebeam). Optionally, the thickness of the powder layer (as viewed from the Ebeam) is configured such that the amount of irradiation received by the front side (source facing) is not more than 25, 50, 75 or 100% greater than the irradiation received by the back side of the powder layer. The packaging may be moved on a conveyor 430 and irradiation preferably occurs in a temperature controlled environment configured to keep the TH Powder at below 50, 37 or 25 degrees Celsius.

### Exemplary tissue processing recipes & conditions according to various embodiments.

Combinations and various of these recipes may be made to achieve alternative embodiments.
(1) Collecting, shipping and preparing the raw placental tissue: collect the porcine placentas within 2 hours after the delivery, remove debris from the placentas, immerse in antiviral antimicrobial disinfectant 0.01 - 1% peracetic acid for 2 hours, freeze, cold chain delivery shipping in ^{~}-10 Degrees Celsius, weigh, wash, inspect tissue, and remove umbilical cord, amniotic membrane and large blood vessels.
(2) Viral inactivation: a working solution with a concentration range of 0.15%-0.3% peracetic acid and 2%-3.5% ethanol.
(3) Decellularization:
   multiple freeze-thaw cycles, e.g., 2, 3, 4 or more;
   50mM hypertonic solutions (50mM Tris-HCl, 2M NaCl, pH^{~}8.0);
   1% Triton X-100, 0.1 mM phenylmethylsulfonyl fluoride (PMSF), 0.1% EDTA;
   10mM hypotonic solution (10mM Tris-HCl, pH^{~}8.0);
   2.5% sodium deoxycholic acid;
   100 U/mL DNase I, 20mM magnesium chloride (MgCl₂), 0.1 mM PMSF;
   10mM EDTA; and
   a one or more cycle of freeze and thaw.
(4) 1^{st} Lyophilization by using industrial size lyophilizers with multiple temperature programming.
(5) 1^{st} Cryogrinding using the novel cryogrinding methods and systems illustrated in FIGs. 2 and 3.
(6) Digestion and solubilization: dissolve porcine pepsin with a digestion working solution in a shaker for 24-36 hours and produce a tissue derived hydrogel.
(7) Neutralization, self-assembly cross-linking and polymerization: neutralize the hydrogel to reach a pH in the range of 6.5 to 7.2 (<7.2).
(8) Adding mixture: adding preservative and/or therapeutic materials.
(9) 2^{nd} Lyophilization by using industrial size lyophilizers with multiple temperatures programed.
(10)2^{nd} Cryogrinding using the novel cryogrinding methods illustrated in FIGs. 2 and 3.
(11)Selecting particle sizes less than 200 µm, e.g., using a mesh.
(12)Packaging with the novel carton packaging system illustrated in FIG. 4.
(13)Freezing at ^{~} -18 Degrees Celsius for 48 hours.
(14)EB irradiation sterilization with a dose range of 15 ^{~} 25 kGy.
(15)Rehydrate with saline shortly prior to application.

The uses of the detergents and working solutions:
(1) Peracetic acid and ethanol: viral inactivation.
(2) Hypertonic and hypotonic solutions: cell lysis by osmotic shock, disrupt DNA-protein interactions.
(3) Triton X-100: disrupts DNA-protein interactions, disrupts lipid- lipid and lipid-protein interactions and to decrease protein-protein interactions.
(4) Sodium deoxycholic acid: solubilizes cell and nucleic membranes, denatures proteins.
(5) DNase I: digests single or double stranded DNA to produce single or double stranded oligodeoxynucleotides.
(6) MgCl₂: as a cofactor, activates DNA enzyme activity.
(7) PMSF: inhibits many proteases to maintain the native extracellular matrix ultrastructure.
(8) EDTA: chelate agents bind together metallic ions, thereby disrupt cell adhesion to ECM. Chelate divalent metal ions, which help cells separate from tissue proteins by isolating metal ions and inactivating the remaining DNA enzymes.

In an illustrative procedure, which may be performed in conjunction with the methods and/or systems illustrated in FIGs. 1, 2, 3 & 4:

In the Prepare Tissue Step 115, porcine placental tissue is washed to remove unwanted matter. The materials removed can include umbilical cord and impurities, some large blood vessels, blood fluid and amniotic tissue until no foreign matters or blood vessels are visible. This step may include washing 3 times with PBS. Multiple freeze-thaw cycles at -80^{~}25 Degrees Celsius, to damage cell membranes. In the flow of DI water, rub the tissue repeatedly with a mesh for 5 min, carefully remove blood vessels and dirty tissue. Rinse the tissue with DI water in a shaker (room temperature, 140-160 rpm) for 30 min.

In the Decellularization Step 125, in a flow of DI water, rub the tissue repeatedly with a mesh for 5 min, carefully remove blood vessels and dirty or sullied tissue. Rinse the tissue with 0.05% Trypsin / 0.02% EDTA solution in a shaker (room temperature, 140-160 rpm) for 120 min. In the flow of DI water, rub the tissue repeatedly with a mesh for 5 min, carefully remove blood vessels and dirty or sullied tissue. Rinse the tissue with 2.5% Triton X-100 solution in a shaker (room temperature, 140-160 rpm) for 120 min. In the flow of DI water, rub the tissue repeatedly with a mesh for 5 min. Rinse the tissue with 3% sodium deoxycholic acid solution in a shaker (room temperature, 140-160 rpm) for 120 min. In the flow of DI water, rub the tissue repeatedly with a mesh for 5 min. Put the tissue in 1L beaker containing PBS. Stored at 4°C for 60 min. In the flow of DI water, rub the tissue repeatedly with a mesh for 5 min. Add a solution of 0.15% - 0.3% peracetic acid and 2% - 3.5% ethanol working solution for a minimum of 120 min exposure time to remove residual nucleic acid and viral inactivation. Rinse the tissue with 1% Triton X-100 in a shaker (room temperature, 140-160 rpm) for 20-60 min. In the flow of DI water, rub the tissue repeatedly with a mesh for 5 min. Rinse the tissue with PBS in a shaker (room temperature, 140-160 rpm) for 30 min. Freeze the tissue in -80 Degrees Celsius refrigerator for 6 hours, dissolve in running water and rub the tissue repeatedly with a mesh for 60 min (Method 1). One or more of these steps are optionally automated.

In alternative embodiments, the decellularization can include rinsing the tissue with 50mM hypertonic solution in a shaker (room temperature, 140-160 rpm) for 60 min. In the flow of DI water, rub the tissue repeatedly with a mesh for 5 min. Rinse the tissue with a buffer solution (1% Triton X-100; 0.1 mM PMSF; 0.1% EDTA) in a shaker (140-160 rpm) for 60 min. In the flow of DI water, rub the tissue repeatedly with a mesh for 5 min, tissue is then rinsed with a 10mM hypotonic solution on a tissue cleaning device (140-160 rpm) for 60 min. In the flow of DI water, rub the tissue repeatedly with a mesh for 5 min. The tissue is again rinsed with 2.5% sodium deoxycholate solution in a shaker (room temperature, 140-160 rpm) for 60 min. Again, rinse the tissue with a buffer solution (1% Triton X-100; 0.1 mM PMSF; 0.1% EDTA) in a shaker (room temperature, 140-160 rpm) for 60 min. In the flow of DI water, rub the tissue repeatedly with a mesh for 5 min, carefully remove any remaining tissue debris. Rinse the tissue with a DNase I solution (100 U/mL DNase I; 20mM MgCl₂; 0.1 mM PMSF) in a shaker (room temperature, 80^{~}110 rpm) for 10 hours. In the flow of DI water, rub the tissue repeatedly with a mesh for 5 min and remove any tissue debris. Rinse the tissue with an EDTA solution (10 mM EDTA) in a shaker (room temperature, 140^{~}160 rpm) for 60 min. In the flow of DI water, rub the tissue repeatedly with a mesh for 60 min. Freeze the tissue in - 80 Degrees Celsius refrigerator for 6 hours. Dissolve in running water and rub the tissue repeatedly with a mesh for 60 min (Method 2).

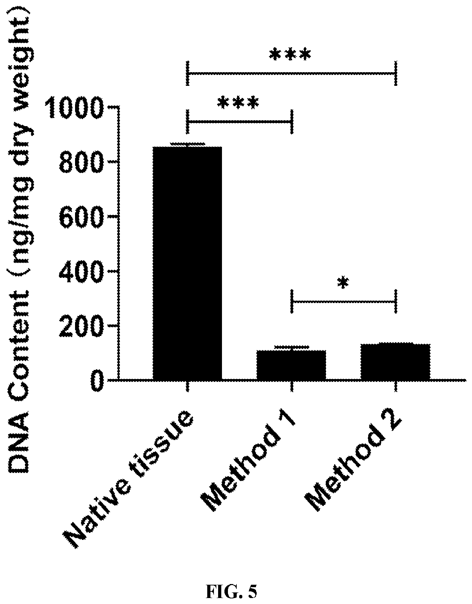

FIG. 5 illustrates the DNA content of the native tissue and the decellularized tissue processed by the two exemplary decellularization methods above. **p <* 0.05, ****p <* 0.001.

### Exemplary Applications.

As described herein, various embodiments of the invention include making a micronized and lyophilized decellularized tissue matrix derived powder (TH Powder) which can be rehydrated to a flowable and injectable tissue derived hydrogel or a liquid. Other mixture materials, such as preservatives, mucopolysaccharides, PEG, and additional tissue, can be added to the powder. The resulting materials (powder, paste, hydrogel and solution) can be used in various therapeutic and non-therapeutic applications. In these applications the resulting materials may provide an extracellular matrix 3D scaffold and a moist environment to promote wound healing, a barrier to minimize the formation of scars, adhesions or fibrosis, a temporary cover to minimize the irritation, or function as a bio-glue to adhere the membranes together. Also, a carrier matrix can be configured by rehydrating the TH Powder directly with the liquid pharmaceutical drugs or therapeutic agents in Step 190. Optionally, the pharmaceutical drugs or therapeutic agents can be added in Step 145, or added to the grinding tank in Step 160. This enables the use of the TH Powder as carrier matrix in many clinical applications.

As discussed elsewhere herein, if the particle size is small and the concentration is high, the solubility of the rehydrated TH Powder is increase, the viscosity and adhesiveness is also increased, and the polymerization and solidification time is shortened. In some embodiments, it takes only 3-5 minutes to polymerize and solidify (at average normal human body temperature) for the rehydrated TH Powder with a concentration of 30mg/mL or higher and a particle size smaller than 200 µm.

The environment in which different tissue and organs are located in the human body is also very different. Different concentrations of TH Powder affect the final degradation rate of the rehydrated TH Powder. A higher concentration results in a longer biodegradation time compared to a lower concentration.

As discussed elsewhere herein, the TH Powder can carry additional components (other than tissue) including pharmaceutical drugs or therapeutic agents for sustained release. Also, the TH Powder can directly mix with liquid pharmaceutical drugs or therapeutic agents. Some of these agents, for example, stem cells, are optionally added after sterilization to preserve the function of biological species. The rehydrated TH Powder, of various embodiments of the invention, can carry a variety of the components, such as preservatives, pharmaceutical agents, exosomes, anti-inflammatory drugs, antioxidants, and the like. These components can be used alone or in combination with each other. Additional examples of materials that may be included in the rehydrated TH Powder is listed in Table 5.

**Table 5**

| Classification | Function | Examples |
|---|---|---|
| Nonsteroidal Anti-inflammatory Drugs | • Anti-inflammatory | • Acetylsalicylic acid |
| | • Antipyretic analgesia | • Paracetamol |
| | • Reduce the side effects of these drugs on the gastrointestinal | • Ibuprofen, Diclofenac |
| | | • Indomethacin |
| | | • Piroxicam |
| | | • Antioxidants |
| | | • Cannabidiol |
| | | • Polyphenols |
| | | • Hydroxytyrosol etc. |
| Stem cells | • Support stemness potential of stem cells | • Embryonic stem cells |
| | | • Mesenchymal stem cells derived from bone marrow, adipose tissue, placenta or umbilical cord, peripheral blood and other tissues. |
| | • Maintain stem cell proliferation and differentiation after transplantation | |
| | • Enhanced transient paracrine actions of stem cells | • Intestinal stem cells |
| | | • Hematopoietic stem cells |
| | | • Epidermal stem cells etc. |
| Antibiotic | • Reduce the number of pathogenic bacteria in tissues and organs or body cavities | • β-Lactam antibiotics |
| | | • penicillin |
| | | • Cephalosporins |
| | | • Tetracycline antibiotics |
| | | • Aminoglycoside antibiotics |
| | | • Macrolide antibiotics etc. |
| Anti-metabolites | • Reduce scar formation | • 5-Fluorouracil (5-FU) |
| | • Reduce adhesion formation | • Mitomycin C (MMC) etc. |
| Exosomes | • Maintain stabilization of exosomes in vivo and in vitro. | • placenta mesenchymal stromal cells exosomes |
| | • Maintain slow release of exosomes in vivo. | • adipose-tissue extracellular vesicles |
| Vitamins | • Maintain slow release of vitamins in vivo. | • Vitamin E |
| | | • Vitamin B |
| | | • Vitamin C |
| | | • Vitamin A etc. |
| Hemostatic drugs | • Enhanced coagulant factors activities | • Vitamin K1, K3, K4 |
| | | • Vitamin C, Carbazochrome Sodium Sulfonate, Adrenochrome semicarbazone |
| | • Reduced capillary | |
| | permeability | |
| | • Blocked fibrinolysis | • Aminocaproic Acid, Aminobenzoic acid |
| | | • Tranexamic acid etc. |
| Chemotherapy drugs | • reduce new growth of malignant (cancerous) cells and limit recurrences | • Alkylating agents |
| | | • Nitrosoureas |
| | | • Antimetabolites |
| | | • Anti-tumor antibiotics, |
| | | • Topoisomerase inhibitors |
| | | • Mitotic inhibitors |
| | | • Corticosteroids |
| | | • Other chemotherapy drugs |
| Anti-VEGF drugs | • Anti-neovascularization | • Bevacizumab |
| | | • ranibizumab |
| Growth factor drugs | • stimulating and growth factors to increase the cell production and tissue growth | • recombinant epidermal growth factor, human (hEGF) |
| | | • Leukocyte Growth Factor |
| Other pharmaceutical agents | Analgesics, immunomodulators, hormones/neurotransmitters and other bioactive substances | |

In various embodiments, human and/or animal tissue used to produce the TH Powder is selected for inclusion in the composition based on anti-inflammatory compounds and/or effects found within the tissue. In some embodiments, the TH Powder includes a combination of compounds that reduce immune response (e.g., have an anti-inflammatory effect) and antibiotics or fungicides that reduce bacterial or fungal infection. For example, for conditions such as dental surgery or puncture wounds, the TH Powder can result in a decrease in T-cell response while an (optionally included) antibiotic reduces likelihood of infection. The TH Powder provides less inflammation and decrease chances of infection at the same time.

Inflammation has its positive side, but excessive inflammation can slow wound healing and induce formation of scar, adhesion and fibrosis. The rehydrated TH Powder containing possible pharmaceutical agents are optionally configured to target excessive inflammation and minimize the formation of scar, adhesion and fibrosis. These properties in the TH Powder may be related to the immune privileged characteristics of the placenta. The preparation methods of the TH Powder discussed herein preserve such biological properties of the original tissue. Specific characterization of excessive inflammation may include degrees and time of infiltration of inflammatory cells and the levels and types of inflammatory cytokines release.

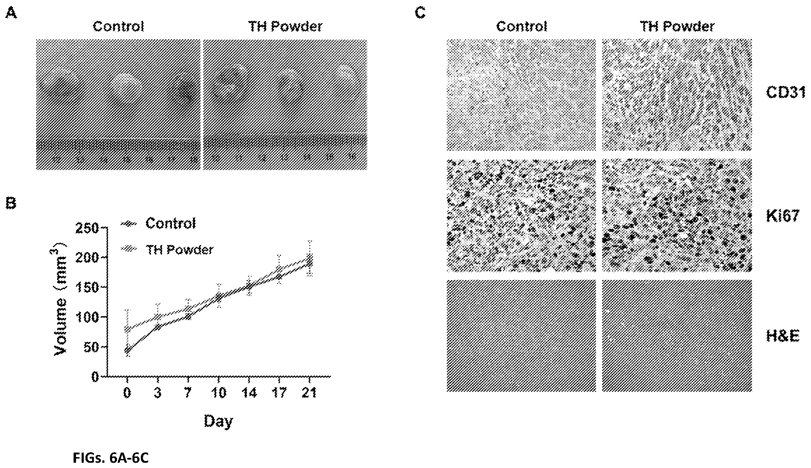

FIGs. 6A-6C illustrate the TH Powder as evaluated in a mouse xenograft model of breast cancer (4T1) to determine effect on tumor growth. The TH Powder has no influence on cell-line derived xenograft model. The mouse breast cancer cell line (4T1) was cultured in RPMI-1640 medium containing 10% FBS at 37 Degrees Celsius. After digestion with 0.25% trypsin, the cells were collected and counted, and the cell concentration was adjusted to 5×10⁷/mL and injected into the back of nude mouse with 0.1 mL each mouse. TH Powder was rehydrated with normal saline to form a hydrogel, which was injected into mouse tumor site once a week.

FIG. 6A illustrates macrographs of tumor in a control group and a TH powder group on day 21.

FIG. 6B illustrates tumor size in the control group and the TH powder group. There were no significant differences between two groups. All tumor sizes of mice were calculated by long diameter (mm) × short diameter² (mm) /2 every three days.

FIG. 6C includes typical pathological images (including CD31, Ki67 and H&E staining) of tumors in the control group and the TH powder group and there were no significant differences between the TH powder group and the control group.

Briefly, tumor-burdened mice were treated with the TH Powder via subcutaneous injections once weekly until endpoint (Day 21). There were no significant differences between the saline and the TH Powder treated mice with regards to tumor volume, proliferation (Ki-67), inflammation, angiogenesis (CD31), or apoptosis (TUNEL). We concluded that the TH Powder is completely neutral with regards to tumor growth and kinetics. Thus, we tend to believe with reasonable certainty that it is safe to apply the TH Powder as a gel, a spray or an enema irrigation directly on to the skin or mucosal membrane defects where the cancerous cells are present, which include irradiation or chemotherapy induced wounds such as dermatitis, oral mucositis, stomatitis, esophagitis, proctitis, urethral irrigations, vaginitis, enteritis, brain necrosis and the like.

The methods of using the TH Powder and the various rehydrated forms of the TH Powder including a hydrogel, a paste, a gel, a spray, an enema irrigation, a solution or a drug carrier of various embodiments of the invention may be varied depending on the specific ailment being treated. In Apply Step 195, the TH Powder can be directly applied onto a wound or injury. The application methods can vary significantly depending on the nature of the wound or injury, and the desired therapeutic effect. For example, the rehydrated TH Powder can be injected into postoperative cavities, applied onto the skin and then covered with a dressing, applied as part of a wound dressing, applied as a coating on sutures, applied on a surface of a device to be implanted into the body (e.g. deep brain stimulator, knee or hip replacement implants), applied under a vacuum assisted healing device, and/or the like. The dry powder can be directly applied into the postoperative body cavities or external skin defects. The TH Powder can be applied both *in vitro* and *in vivo.*

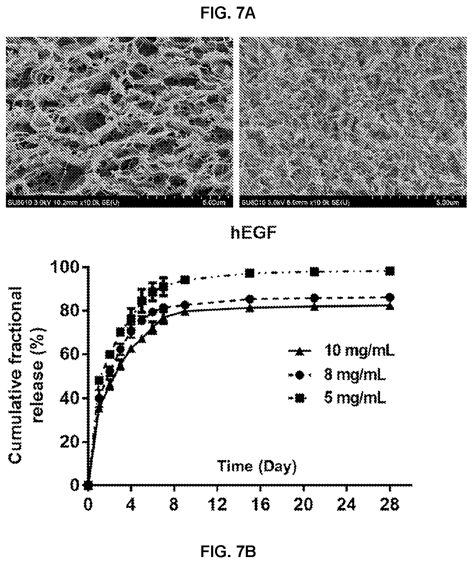

TH POWDER AS A CARRIER. The TH Powder or the rehydrated TH Powder can be used as a carrier containing one or more pharmaceutical drugs or pharmaceutical agents such as recombinant epidermal growth factor, human (hEGF), ranibizumab, bevacizumab, exosomes, and/or any of the other pharmaceutical and/or therapeutic agents discussed herein. As described elsewhere herein, when the various pharmaceutical drugs and therapeutic agents are added in Add Mixture Step 150 or directly in Rehydrate Step 190, the porous structure of the rehydrated TH Powder (or the hydrogel) can trap the drugs or agents) (See, for example, FIG. 7A). At the target site, with the degradation of the TH Powder, the pharmaceutical drugs and therapeutic agents can be released in a sustained period, but not under a constant rate. The sustained release of pharmaceutical drugs or therapeutic agents carried by the TH Powder can last from a few weeks to several months in a concentration-dependent manner (See FIG. 7B).

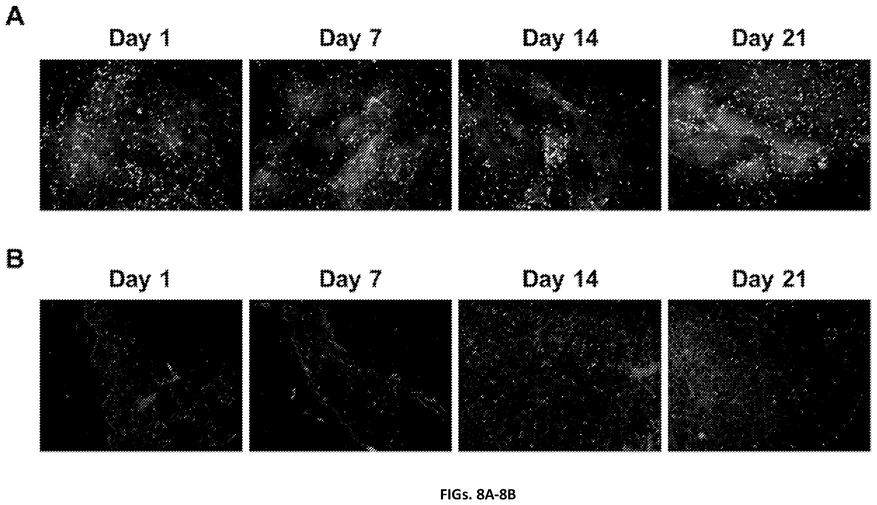

TH POWDER MANAGING JOINT INJURY AND JOINT DEGRADATION. The TH Powder (dry or rehydrated) can act as a lubricant in the joint cavity, reduce the friction between the tissue, reduce inflammation, relieve pain, increase joint mobility, and/or possibly regenerate articular cartilage. FIGs. 8A-8B illustrate use of TH Powder evaluated in an in vitro study to determine effect on growth and regeneration of cartilage cells. FIG. 8A illustrates the viability of cartilage cells 3D-cultured in hydrogel on Day 21 was significantly higher than Day 1, determining no adverse effect of the TH Powder on the cell viability, and possible therapeutic potential in cartilage cells proliferation. FIG. 8B illustrates COL II release of cartilage cells 3D-cultured in the rehydrated TH Powder is increased significantly on Day 14 and Day 21, compared to Day 1.

A barrier function of the rehydrated TH Powder can effectively prevent the diffusion of inflammatory mediators and reduce the stimulation of pain receptors by chemical substances and achieve the reduction of joint pain. Examples of therapeutic agents which may be included in the TH Powder for these applications include steroids, anti-inflammatory agents, pain relievers (e.g., opioids), collagen, hyaluronic acid, anti-oxidants, and/or the like. Any of the other therapeutic agents discussed elsewhere herein can be included in the TH Powder for these applications.

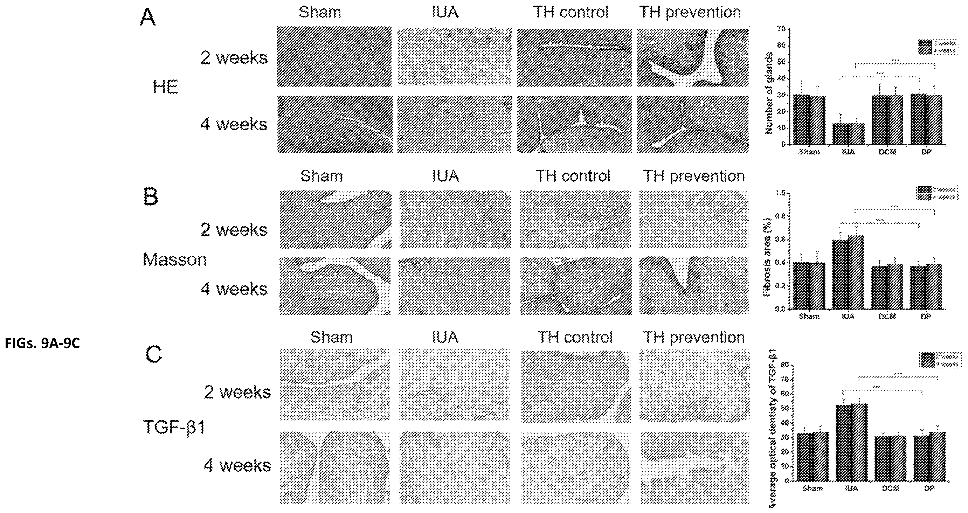

TH POWDER AS A COATING ONTO IMPLANTABLE DEVICES. Implantable devices (for example, heart stent, syringe or catheter) sometimes have risks in localized inflammatory response and in-stent restenosis due to the formation of scars, adhesions and fibrosis. The rehydrated TH Powder has a therapeutic potential to prevent and manage adhesions (FIG. 9), thus it can be applied as a coating onto the stent to prevent and manage in-stent restenosis. In-stent Restenosis (ISR) remains a challenge nowadays. Much effort has been concentrated on finding new methods to prevent scarring by covering the stent pores using films made by various polymers. However, these polymers have been shown to elicit severe inflammatory responses due to low biocompatibility. Covering the stent with the rehydrated TH Powder, which quickly polymerizes and solidifies within 3^{~}5 min, forming a protective coating and covers the stent, not only prevents and manages ISR, and also manages the wounds on the connective tissue and endothelium. The TH Powder is highly biodegradable and biocompatible with humans or animals. The rehydrated TH Powder can also be applied as a coating onto other implantable devices (for example, catheter, drainage tube, pacemaker, peripheral IV lines, arterial lines) to prevent and manage adhesions. Any of the other pharmaceutical drugs or therapeutic agents discussed herein can be included in the TH Powder for these applications.

TH POWDER MANAGING INTRAUTERINE ADHESIONS (IUA). Intrauterine adhesions occur to a high percentage of women who have had multiple curettage (D&C) surgeries. Scar tissue from uterine surgeries like dilation and curettage (D&C) cause more than 90% of IUA. Also, scar tissue from a Cesarean section or from sutures used to stop hemorrhage, endometriosis, infections of the reproductive organs and radiation therapy treatment can cause IUA. When applied in vivo, rehydrated TH Powder quickly polymerizes and solidifies within ^{~}3-5 min at the average normal body temperature and adheres to the endometrium wall, provides a protective layer, prevents scar formation and intrauterine adhesions, and provides a moist environment for intrauterine wound healing. The TH Powder provides anti-inflammatory effect and enhances the regeneration of the endometrium and glands, and thus may improve fertility and pregnancy outcomes.

TH POWDER MANAGING OCULAR SURFACE INJURIES AND DRY EYE. Ocular surface wounds such as corneal ulcerations and abrasions are common ocular injuries, which may result in corneal neovascularization and scarring and then cause serious visual impairment and even blindness. Moreover, once blood vessels are formed, they are hard to remove. Rehydrated TH Powder as eye drops can manage ocular surface wounds, and prevent (or minimize) corneal neovascularization, anti-inflammatory, anti-scarring and anti-angiogenesis. In addition, rehydrated TH Powder can serve as an excellent substitute ocular surface fluid, which makes it suitable for both clinical and home/office use. Given its unique function of self-assembly and cross-linking, the rehydrated TH Powder can quickly form a protective film within ^{~}3-5 min at normal human body temperature, to protect the cornea from external stimulus and provide a 3D scaffold biological environment to manage ocular surface injuries. The TH Powder also demonstrates a strong efficacy in easing the symptoms of dry eye. The animal model of corneal chemical (alkali) burns is often used to evaluate the product efficacy in treating dry eye.

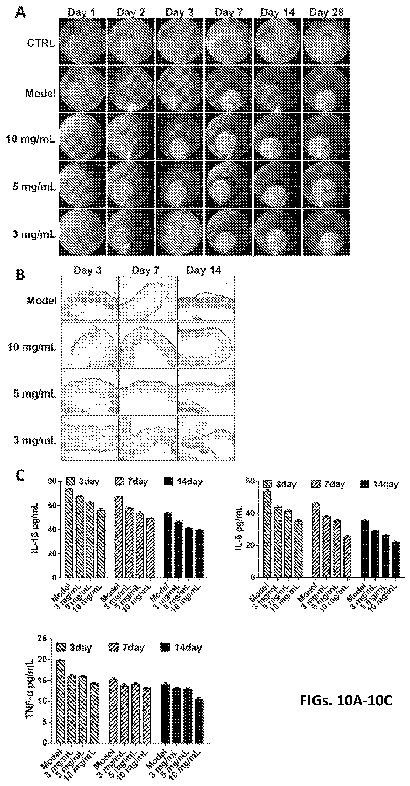

FIGs. 10A-10C Illustrate use of TH Powder for management of ocular surface wounds following injuries and eases dry eye symptoms in a rat corneal alkali burn model treated with 1 M sodium hydroxide, according to various embodiments of the invention. In these figures: Control (CTRL), saline+alkali burn; Model, alkali burn; 10mg/mL, rehydrated TH Powder; 5mg/mL, rehydrated TH Powder; 3mg/mL, rehydrated TH Powder. In FIG. 10A, the 10mg/mL group showed a greater therapeutic potential in managing corneal turbidity and neovascularization than the 5mg/mL, 3mg/mL, control and model group. In FIG. 10B, the corneas were collected on days 3, 7, and 14. The local epithelial detachment was still observed in the model group on the 14^{th} day. The epithelium in the TH Powder treated groups (3mg/mL, 5mg/mL and 10mg/mL) was almost intact. In FIG. 10C, the corneas were collected on days 3, 7, and 14. The content of IL-1β, IL-6, and TNF-α were detected by ELISA. The release of key pro-inflammatory cytokines in corneas, including IL-1β, IL-6, and TNF-α, is decreased significantly after being treated with the TH Powder. The higher concentration of the rehydrated TH Powder demonstrated a stronger therapeutic potential in anti-inflammatory efficacy.

Examples of therapeutic agents which may be included in the TH Powder for these applications include anti-inflammatory agents, anti-oxidants, anti-angiogenesis and a corneal friendly preservative Polyquaterium-1, and/or the like. The rehydrated TH Powder, possibly including therapeutic agents and/or preservatives discussed elsewhere herein, has an osmotic pressure between 200 and 400, which matches the osmotic pressure of the extraocular fluids.

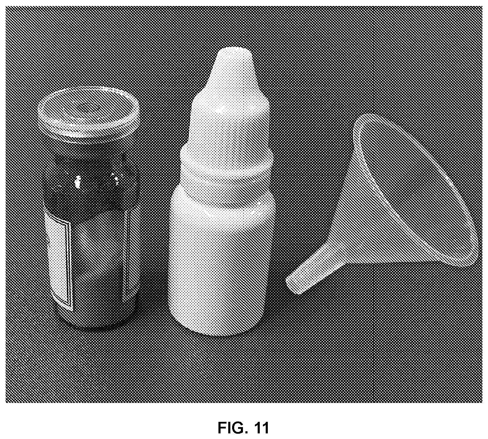

An exemplary ophthalmic treatment composition includes: (1) the rehydrated TH Powder at least 2mg/mL, 5mg/mL, 10mg/mL, 20mg/mL, 30mg/mL, or 40mg/mL or any range between these values; or less than 2mg/mL; (2) saline, for example, ^{~}0.3%-0.9%; (3) an ophthalmic rehydration solution comprising sodium chloride, potassium chloride; for example, sodium chloride at a concentration of at least 0.1% or 0.2%; or between 0.1% and 0.2%; or less than 0.2% (percentages herein are by weight percent); for example, potassium chloride at a concentration of at least 0.02% or 0.05%; or between 0.02% and 0.05%; or less than 0.05%; and (4) an ophthalmic rehydration solution comprising sodium chloride, potassium chloride, and optional polyethylene glycol (PEG), carboxymethylcellulose sodium (CMC-Na) or sodium hyaluronate; for example, PEG at a concentration of at least 0.05% or 2%; or between 0.05% and 2%; or less than 2%; for example, CMC-Na at a concentration of at least 0.05% or 2.5%; or between 0.05% and 2.5%; or less than 2.5%; for example, sodium hyaluronate at a concentration of at least 0.1% or 0.5%; or between 0.1% and 0.5%; or less than 0.5%. Wherein the rehydrated fluid optionally includes purified water and/or liquid pharmaceutical drugs. The powder includes ground tissue as described elsewhere herein and optionally comprising placenta tissue, such as porcine, bovine or human placenta tissue. FIG. 11 illustrates the ophthalmic ocular surface wound treatment composition kit, according to various embodiments of the invention. The kit comprises a glass vial containing the TH Powder, a dropper bottle and a funnel.

TH POWDER MANAGING RETINAL DISEASES. The rehydrated TH Powder can be directly applied as an intravitreal injection, optionally being directly mixed with liquid drugs such as bevacizumab or ranibizumab to treat retinopathy, Age Related Macular Degeneration (AMD), neovascularization and other retinal disease.

AMD is a disease that affects the macular region of the retina, causing progressive loss of central vision. In various embodiments, the TH Powder is a rich reservoir for hundreds of proteins, and a variety of growth factors and cytokines such as epidermal growth factor (EGF), transforming growth factor-β (TGF- β), fibroblast growth factor (FGF), platelet-derived growth factor (PDGF). Thus, the TH Powder may have an excellent potential in slowing degeneration of photoreceptor cells and promoting regeneration of photoreceptor cells, retinal pigment epithelium (RPE) cells and retinal progenitor cells (RPC). These growth factors/cytokines play a significant role in fibroblasts/keratinocytes migration and proliferation, mesenchymal stem cells homing, reepithelialization, which can potentially also manage geographic atrophy (Dry AMD), a retinal disease that is characterized by thinning of retina atrophy and loss of pigmentation. As disclosed elsewhere herein, TH Powder has an anti-inflammatory and anti-neovascularization effect. Thus, the TH Powder has a potential in managing the progression and aggravation of choroidal neovascularization (Wet AMD), which is caused by neovascularization. Optionally, the rehydrated TH Powder carrying drugs such as pegaptanib sodium, bevacizumab, ranibizumab or aflibercept can be applied as an intravitreal injection to manage Wet AMD.

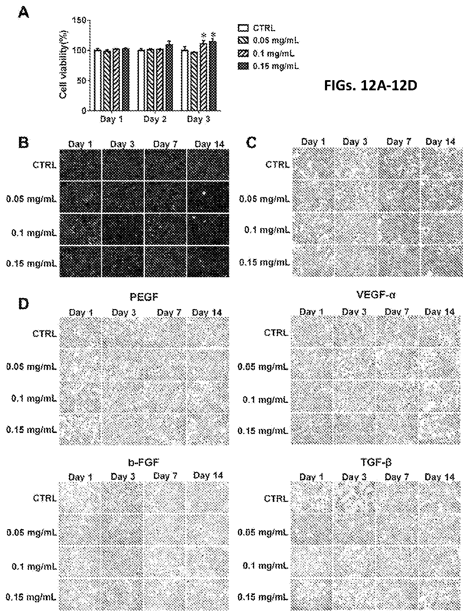

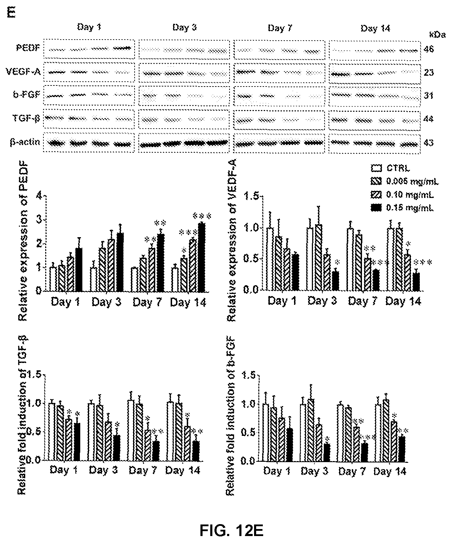

FIGs. 12A-12D illustrate evaluation of TH Powder *in vitro* to determine the viability and anti-angiogenic effect on human retinal pigment epithelial (RPE) cells, according to various embodiments of the invention. Human RPE cells were cultured *in vitro* with the rehydrated TH Powder for 1, 3 and 7 days and 14 days. Three different concentrations of 0.05 mg/ml, 0.10 mg/ml and 0.15 mg/ml rehydrated TH Powder were dissolved in normal medium as three different experimental groups, and normal medium as the negative control group. The normal medium is DMEM medium (containing 10% fetal bovine serum, streptomycin 100 µg/ml and penicillin 100 U/ml). Human RPE cells were cultured in the above four different media *in vitro.* Cytotoxicity test and cell viability assay were done respectively to determine the toxic effect and proliferation effect of the rehydrated TH Powder on the cells. H&E staining showed the morphology of human RPE cells. The *in vitro* secretion of vascular endothelial growth factor-a (VEGF-A), transforming growth factor beta (TGF-β), basic fibroblast growth factor (b-FGF) and pigment epithelium-derived factor (PEDF) were confirmed by H&E stained sections. Western blot experiment confirmed the quantitative expression of VEGF-A, TGF-β, b-FGF and PEDF.

FIGs. 12A-C. illustrate the human RPE cells cultivated in vitro in a higher concentration of the highly diluted rehydrated TH Powder demonstrated a stronger viability.

FIGs. 12D-E. illustrate the sections were stained with H&E to evaluate an anti-angiogenic effect. The expression of the anti-angiogenic factor PEDF in the human RPE cells is increased gradually while the concentration of the rehydrated TH Powder is increased. The expression of angiogenic growth factors VEGF-A, b-FGF and TGF-β gradually is decreased while the concentration of the rehydrated TH Powder is increased (scale bar 100×).

In various embodiments elsewhere herein, TH Powder demonstrates an effect in anti-scarring and anti-inflammatory, which have an excellent potential to be applied during ophthalmic surgeries including cataract extraction, intraocular lens implantation, corneal transplant surgery, glaucoma filtering surgery, trabeculectomy, and/or retina reattachment. TH Powder can also be used as a carrier containing anti-metabolite drug such as 5-fluorouracil (5-FU) or mitomycin C (MMC) and minimize the scar formation after glaucoma surgery. Endophthalmitis is a visually devastating complication of cataract surgery with an incidence ranging from 0.028% to 0.345%. For endophthalmitis treatment, the TH Powder can be used as a carrier containing one or more antibacterial agents such as cefuroxime and delivered onto the ocular surface or applied as an intraocular injection.

To treat retinal tears, retinal detachments and macular holes, vitrectomy is a one of the standard and common surgeries. Often, vitrectomy with air-fluid exchange requires patients to remain in a face-down position all day and night for a period of time varying from days to weeks. Sealing retinal tears/holes and macular holes during vitrectomy or pneumatic retinopexy by applying a material such as glue or film is an excellent and novel approach for preventing vitreous fluid from flowing through the open retinal tears into the subretinal space and can also obviate the time needed for postoperative face-down position. Previously published studies have shown that many adhesives, such as cyanoacrylate, fibrin glue, sodium hyaluronate, carboxymethylcellulose absorbable film, mussel protein, transforming growth factor β, and polysiloxanes, have their own disadvantages, including various degrees of ocular toxicity, weak adhesive force, severe inflammatory response. In view of the above reasons, the use of the adhesives has not yet become a standard procedure in the treatment of retinal tears, retinal detachments and macular holes. A better material that is effective, non-toxic, and easy to use is urgently needed.

In some embodiments, a self-assembly and cross-linking function of the rehydrated TH Powder (>20mg/mL) results from the collagen molecular structure. This structure includes a unique supercoiled triple helix, which does not depend on an exogenous cross-linking agent. When performed under the air bubble, the retina or the macular is exposed and is dry or semi dry Under these conditions rehydrated TH Powder (>20 mg/ml) can be applied onto the retina or macular. The rehydrated TH Powder is adhesive and rapidly adheres to the retina or macular, polymerizes and solidifies within ^{~}3-5 min, quickly sealing retinal tears/holes or macular holes and preventing further expansion. This solidification rate is highly advantageous during surgery and in various embodiments the TH Powder can be configured to solidify within ^{~}3, 5, 7, 10, 12, or 15 minutes. After the vitreous humor is removed, and in some cases the laser surgery (photocoagulation) or freezing (cryopexy) is required in order to further secure the retina to the eye wall, the solidification of the TH Powder is irreversible and sufficient to repair tears and holes. The application of the TH Powder avoids the uncomfortable and challenging postoperative face-down position. Moreover, in contrast with the poor biocompatibility of traditional adhesives, the TH Powder is 100% natural, has excellent biocompatibility and is biodegradable. Moreover, the various proteins and growth factors/cytokines of the TH Powder play an essential role in tissue repair and regeneration of the RPE cells regeneration and may restore the lost vision. The rehydrated TH Powder, possibly including therapeutic agents discussed elsewhere herein, may be configured to have an osmotic pressure around ^{~}300-305, which is compatible with the osmotic pressure of the intraocular fluids.

Optionally, a novel intraocular (or intravitreal) injection treatment method is configured to combine the rehydrated TH Powder in use with polysaccharides, Indocyanine Green (ICG) or "heavy" Brilliant Blue G (HBBG), which are used to weight down the rehydrated TH Powder onto reach to retina. Polysaccharides, ICG and HBBG have higher density and weight than the rehydrated TH Powder.

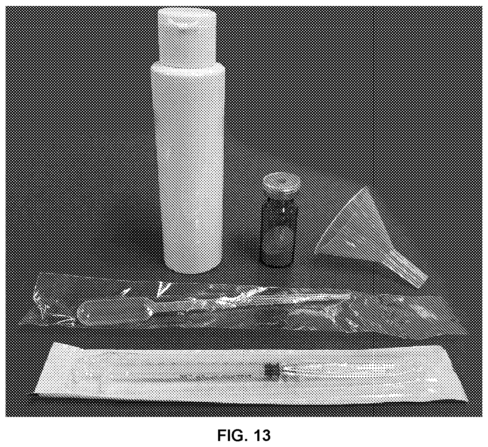

FIG. 13 illustrates the ophthalmic intravitreal treatment composition kit, according to various embodiments of the invention. The kit comprises a glass vial containing the TH Powder, a dropper bottle/mixing bottle, a funnel, a syringe and a mixing rod.

TH POWDER MANAGING VAGINAL, URETHRAL, ANORECTAL OR GASTROINTESTINAL WOUNDS AND IRRITATIONS, AND PELVIC RADIATION INDUCED WOUNDS. The rehydrated TH Powder can serve as a personal lubricant. For example, in these embodiments, the rehydrated may have a pH suitable for penile, vaginal application, and/or anorectal, intended to lubricate and moisturize, to enhance the ease and comfort of intimate sexual activity and supplement the body's natural lubrication. These embodiments are compatible with natural rubber latex and polyisoprene condoms. An exemplary formulation that may be used as a personal lubricant includes: (1) the rehydrated TH Powder at a concentration of at least 2mg/mL, 5mg/mL, 10mg/mL, 20mg/mL or 30mg/mL, or any range between these values; or less than 2mg/mL; (2) PEG at a concentration of at least 0.05%, 1%, 5%, 10%, 15% or 20%, or any range between these values; or less than 20%; (3) optional hyaluronic acid at a concentration of at least 0.05%, 1%, 5%, 10%, 25%, 50% or 75%, or any range between these values; or less than 75%; (4) optional hydroxyethylcellulose at a concentration of at least 0.05%, 1%, 5%, 10%, 25%, 50% or 75%, or any range between these values; or less than 75%; (5) optional preservatives such as potassium sorbate; (6) optional saline solution; (6) optional vitamins such as Vitamin C, Vitamin E, Nicotinamide also known as Vitamin B3, Vitamin B12; (7) optional flavoring or scent agent; (8) optional food grade colorants; (9) optional antiviral agents, and/or (10) optional nicotinamide as a whitening agent for the external genitalia.

The TH Powder may be directly applied as dry powder to vaginal or anorectal wounds. Optionally, the rehydrated TH Powder can be delivered as an enema irrigation through an applicator, a syringe, a catheter, or an irrigation device, to manage urethral, vaginal, anorectal or gastrointestinal wounds and irritations, which are not limited to abrasions, surgery sites, ulcers and stenosis, possibly caused by abrasions, trauma, ulcerations, surgeries and cancer treatment pelvic radiation. In these applications, the TH Powder may carry spermicides, anti-viral, and/or antibacterial agents. Radiation vaginitis, proctitis and enteritis often occur during pelvic radiotherapy. The rehydrated TH Powder shows a promising efficacy in preventing and managing these injuries, optionally in use with dilators to manage vaginal and anal stenosis. The rehydrated TH Powder forms a protective layer on the vaginal and anorectal wall, and provides a moist environment for the wound healing.

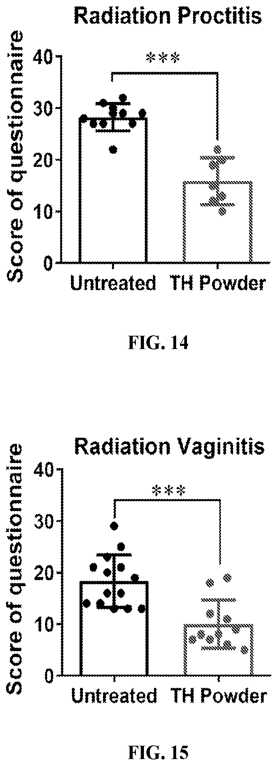

FIG. 14 illustrates clinical case study in cancer patients with radiation induced proctitis, according to various embodiments of the invention. The rehydrated TH Powder was applied as an enema irrigation for 7 consecutive days. Likert scores for radiation proctitis case study ***P<0.001, n=18. FIG. 15 illustrates clinical case study in cancer patients with radiation induced vaginitis, according to various embodiments of the invention. The rehydrated TH Powder was applied as an enema irrigation for 7 consecutive days. Likert scores for radiation vaginitis case study ***P<0.001, n=25. In both FIGs. 14 and 15, patients were asked about the results of their treatment positive responses, e.g., irritation, appearance, pain, and/or discomfort are represented by "score of questionnaire."

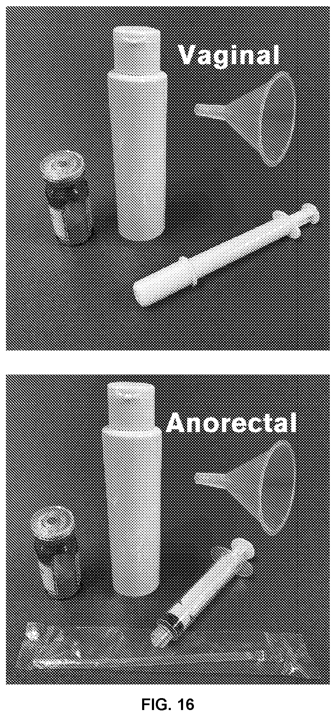

FIG. 16 illustrates the vaginal and anorectal wound care treatment composition kit. The kit comprises a glass vial containing the TH Powder, a mixing bottle, a funnel, an applicator/syringe and optionally a silicon tubing.

TH POWDER MANAGING SKIN WOUND, ORAL AND ESOPHAGEAL DISORDERS AND CHEMORADIOTHERAPY INDUCED GI TRACK WOUNDS. The TH Powder can be used as a dry powder or a rehydrated gel or spray to heal wounds and relieve pain and inflammation in patients with skin, nasopharyngeal, and/or GI Track mucosal membrane wounds. The TH Powder can be used as a dry powder, a rehydrated mouthwash or an oral/nasal spray to heal wounds, relieve the pain and manage inflammation in oral ulcers/sores, gingivitis, post-tooth extraction and the like. The rehydrated TH Powder can also be used as an oral solution to manage oral mucositis, stomatitis, esophagitis and other GI track injuries induced by chemoradiotherapy. In addition, the rehydrated TH Powder can be used to treat the esophagus injuries caused by acid reflux, ulcers, sores or chemical burns. In embodiments in which oral administration is possible, the TH Powder can be applied as dry powder or a rehydrated paste/hydrogel into the tooth cavity to promote oral wound healing and promote tooth bone regeneration and growth. The TH Powder also can be rehydrated into a drinkable liquid or an oral spray.

The added mixtures include vitamins such as Vitamin C, E and B12, and flavoring, for example, strawberry, peppermint or cinnamon flavor. The treatment composition may also include antibiotics, anti-inflammatory agents, antioxidants, flavoring compounds, cytokines, stem cells, antibodies, bacteriophage, and/or any combination thereof.

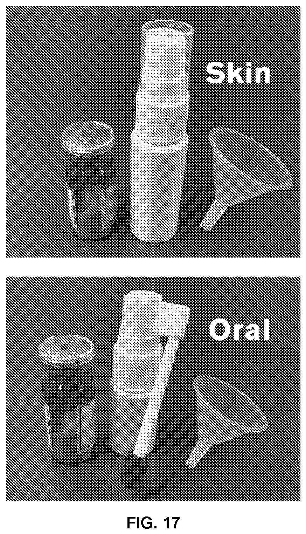

FIG. 17 illustrates the skin and oral wound care treatment composition kit, according to various embodiments of the invention. The kit comprises a glass vial containing the TH Powder, a spray bottle and a funnel.

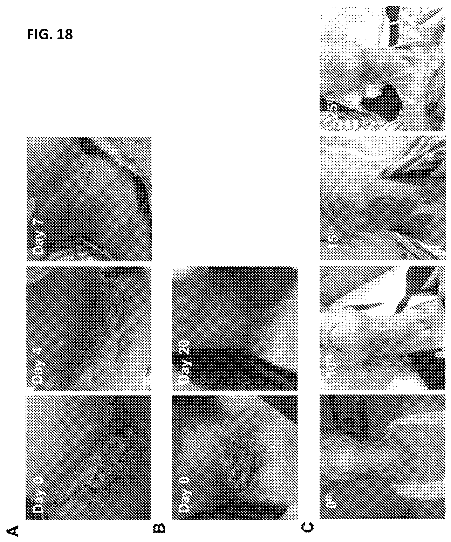

FIGs. 18A-18C illustrate clinical case studies of patients experiencing chemoradiotherapy dermatitis and using TH Powder for the management of dermatitis and pain, according to various embodiments of the invention. In FIG. 18A, a woman with a history of nasopharyngeal carcinoma was treated with radiotherapy combined with chemotherapy (paclitaxel). Skin lesions occurred at the 26^{th} radiotherapy, and ulceration occurred at the 28^{th} radiotherapy. The patient had Grade III radiation dermatitis according to the Radiation Therapy Oncology Group (RTOG) Criteria. The rehydrated TH Powder was applied on Day 0, and radiation dermatitis was significantly alleviated on Day 4 and mostly healed on Day 7. In FIG. 18B, a man with a history of lung cancer was treated with chemoradiotherapy. Skin lesions occurred at the 15^{th} radiotherapy and Grade III radiation dermatitis occurred at the 25^{th} radiotherapy according to the RTOG Criteria. The rehydrated TH Powder was applied at Day 0 and the radiation dermatitis was significantly alleviated at Day 7. In FIG. 18C, a man with a history of larynx cancer was treated by radiation therapy with a linear accelerator and the rehydrated TH Powder applied to the irradiated site successfully prevented radiation dermatitis.

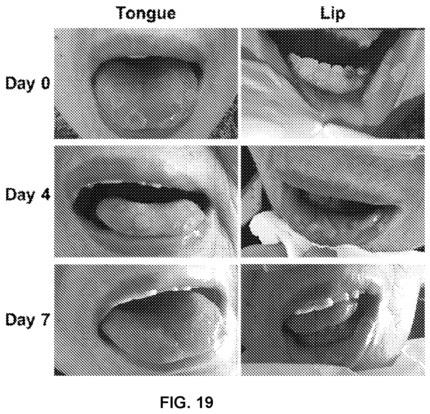

FIG. 19 illustrates a clinical case study of a patient experiencing chemoradiotherapy oral mucositis and using the TH Powder for the management of mucositis and pain, according to various embodiments of the invention.

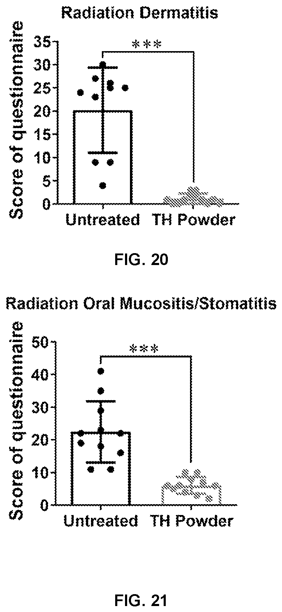

FIG. 20 illustrates clinical case study in cancer patients with radiation induced dermatitis, according to various embodiments of the invention. The rehydrated TH Powder was applied as a spray for 7 consecutive days. Likert scores for radiation dermatitis case study ***P<0.001, n=22.

FIG. 21 illustrates clinical case study in cancer patients with radiation induced oral mucositis/stomatitis, according to various embodiments of the invention. The rehydrated TH Powder was applied as a spray for 7 consecutive days. Likert scores for radiation oral mucositis/stomatitis case study ***P<0.001, n=22.

TH POWDER AS A 3D PRINTING LIQUID. The rehydrated TH Powder can be used as 3D printing material because it quickly polymerizes and solidifies within ^{~}3-5 min at normal human body temperature. In other embodiments of the invention, agents such as polysaccharides or a light activated cross-linking agents may be added to enhance the viscosity and shorten polymerization and solidification time. In various embodiments of the invention, polymerization and solidification of the TH Powder may be initiated and/or accelerated by rehydration, normal body temperature, exposure to air, light (e.g., UV curing) or mixing with an activation agent.

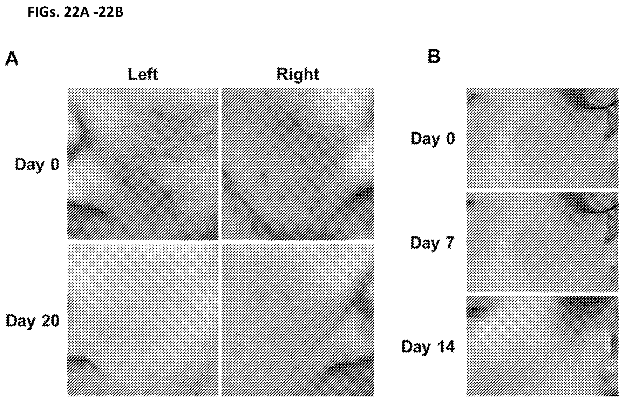

TH POWDER AS A COSMETIC AND AESTHETIC TREATMENT. The rehydrated TH Powder can be used as an ingredient of daily skin care products and can effectively maintain skin moisture, be quickly absorbed by the skin, provide a protective layer against external irritations, reduce local inflammation and promote healing of opened comedo (blackheads) and acne. FIGs. 22A and 22B illustrate clinical case studies in patients with acne vulgaris, according to various embodiments of the invention. Following treatment with rehydrated TH Powder, pustules and cysts were significantly diminished at Day 20 after applying the rehydrated TH Powder daily as a facial serum for 20 consecutive days as shown in FIG. 22A. Inflammation and redness were significantly alleviated at Day 14 after applying the rehydrated TH Powder daily as a facial serum for 14 consecutive days, as shown in FIG. 22B.

In addition, the rehydrated TH Powder accelerates the process of healing during acne treatment, or a post treatment for laser, micro needling and tattoo removal procedures, by reducing inflammation and promoting regeneration of new skin. Any of the other therapeutic agents discussed elsewhere herein can be included in the TH Powder for these applications.

The rehydrated TH Powder has been tested as a subdermal injectable for filling (removing wrinkles) and volumizing the skin. The rehydrated TH Powder can polymerize and solidify underneath the skin and last for ^{~}3-4 months.

The rehydrated TH Powder can be applied during pregnancy to prevent the formation of striae gravidarum by providing collagen and proteins to the damaged skin and collagen fibers. The main treatment for postpartum striae gravidarum is fractional laser treatment. The rehydrated TH Powder can be used in combination with laser treatment, not only promoting the repair of epidermal damage, but also further filling the missing collagen fibers in the depression and promoting muscle fiber regrowth.

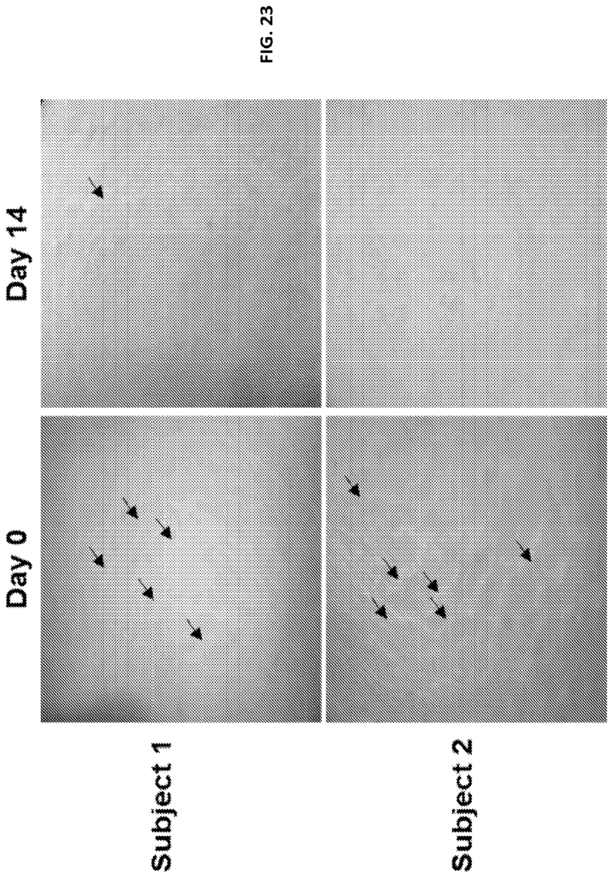

FIG. 23 illustrates clinical case studies in patients with striae gravidarum, according to various embodiments of the invention. On Day 0 the patient was treated with laser, and rehydrated TH Powder was applied twice daily after the laser treatment for 14 consecutive days. Striae gravidarum in two subjects were significantly lightened at Day 14. Black arrows indicated striae gravidarum. Similar TH Powder applications may be used following laser treatment for tattoo removal, pigment modification, treatment of "port wine stains," hair removal, hair restoration, skin exfoliation, or any other dermatological application of lasers.

Under normal circumstances, the skin care products are blocked in the cuticle as a first line of defense. The so-called macromolecules of added nutrients or functional ingredients in skin care products cannot penetrate the stratum corneum or be absorbed by the skin. Mesotherapy, which is the injection of substances locally into mesodermally derived subcutaneous tissue, developed from empirical observations of a French physician in the 1950s. The TH Powder can also be injected into dermis and can quickly eliminate wrinkles, enhance skin's ability to retain water, and improve skin quality. In other embodiments of the invention, the high solubility, flowability and injectability of the rehydrated TH Powder which has small particle sizes allow injections via 25-34 Gauge needles. In addition, the TH Powder can serve as a delivery system carrying bioactive materials, including vitamins, antioxidants, exosomes, stem cells, etc., to enhance its anti-oxidation, anti-aging, anti-wrinkle and moisturizing properties. As a result, the TH Powder can quickly eliminate wrinkles, deeply hydrate skin, minimize brown spots, hyperpigmentation and skin redness, and provide skin filling and volumizing. The mesotherapy result can last up to ^{~}4-6 weeks.

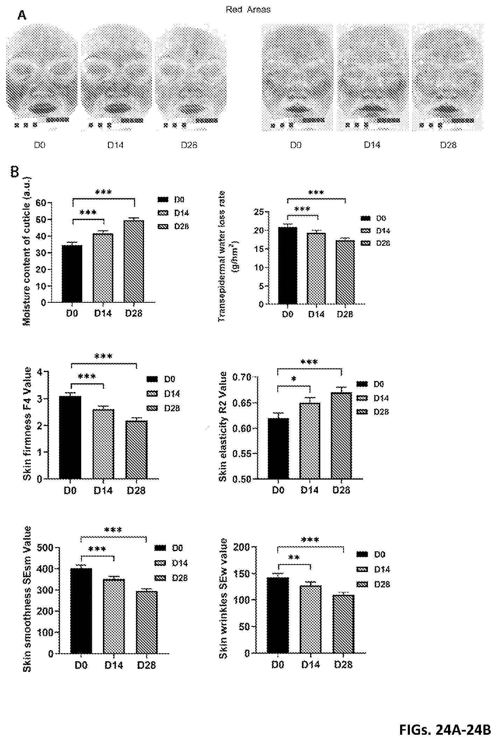

FIGs. 24A and 25B illustrate a 33 subject (compared to the baseline), single center, randomized clinical trial to determine TH Powder efficacy in 11 clinical claims in moisturizing, anti-aging, improving elasticity and anti-wrinkle, according to various embodiments of the invention.

| Test Item | Equipment | Explanation of the Test Item |
|---|---|---|
| Moisture content of cuticle | Corneometer^{®} CM 825 | The higher the measurement value, the higher the moisture content of cuticle. |
| Transepidermal water loss rate | Tewameter^{®} TM Hex | The higher the measurement value, the higher transepidermal water loss rate at the unit interval and unit cross sectional area. |
| Skin fineness | Cutometer^{®} dual MPA 580 | The lower the F4 value, the firmer the skin. |
| Skin elasticity | Cutometer^{®} dual MPA 580 | The higher the R2 value, the more elastic the skin. |
| Skin wrinkles and smoothness | Visioscan^{®} VC 20plus | The lower the SEw value, the fewer the wrinkles. |
| | | The lower the SEsm value, the better the smoothness |

In FIG. 24A, two subjects were treated with the TH Powder. The FIG. demonstrated that the treatment reduced the skin redness on Day 14 and Day 28. In FIG. 24B, statistical graphs are shown of TH Powder treatment efficacy on moisture content of cuticle, transepidermal water loss rate, skin fineness, skin elasticity, skin wrinkles and smoothness after treated on Days 0, 14 and 28.

Cryogenic grinding is described as grinding of biomaterials, such as collagen, fiber or tissue. In alternative embodiments, it can be used in freeze-dry or room temperature grinding applications, such as: (1) environmental agriculture industry: plant seeds (rice, wheat, corn, soybeans, cannabis, etc.), fast iron-free grinding of rhizomes and leaves; (2) electronics and materials industry: mechanical alloying, synthesis of amorphous materials, preparation of high-entropy alloys; (3) Chinese and western medicine: medicinal materials (medlar, rehmannia glutinosa, herbs, honeycomb, powder, etc.) are routinely ground to break down cell walls; (4) textile and paper industry: conventional grinding of multifiber (cotton, linen, paper, cloth, etc.) items; (5) chemical and pharmaceutical industry: constant temperature ball-milling solid-state reaction, conventional rapid mixing and milling of the materials; and (6) animal feed industry: components (bone meal, fish meal, forage, etc.) are ground and mixed. Each of these applications may benefit from the preservation of chemical or biological properties in the ground material.

The embodiments discussed herein are illustrative of the present invention. As these embodiments of the present invention are characterized, various modifications or adaptations of the methods and/or specific ingredients are illustrated. Hence, these descriptions and drawings should not be considered in a limiting sense, as it is understood that the present invention is in no way limited to only the embodiments illustrated.

In exemplary embodiments, the particle sizes below may be used for various clinical indications:

| **Numbers** | **Clinical Applications** | **Mesh** | **Particle D50 Microns** | **Syringe Gauge** | **Solubilizing Time from powder to reconstituted hydrogel/liquid** |
|---|---|---|---|---|---|
| 1 | orthopedic treatments such as intra-articular, tendon, bursa or joint injections | 20 Mesh | 850 microns | 12G | 5-10 min |
| 2 | Topical wound care such as chronic or acute wound management; oral, esophageal, vaginal, anorectal or GI track wound management; ocular surface wound management; intrauterine or abdominal antiadhesion care | 30 Mesh | 600 microns | Used as a powder, a spray, an irrigation or a drop. Syringe is not Applicable | 5-10 min |
| 3 | Intraocular and intravitreal injections | 40 Mesh | 425 microns | 22G | 5-10 min |
| 4 | Intradermal or subcutaneous injections | 50 Mesh | 300 microns | 30G | 5-10 min |

## Claims

1. A method of making a therapeutic material, the method comprising:
obtaining (110) porcine placental tissue having been washed for at least 2 hours in an antibacterial or antiviral solution prior to freezing;
decellularizing (125) the tissue;
lyophilizing (130) the decellularized tissue;
cryogenic grinding (135) the lyophilized tissue at a low temperature of less than 0 degrees Celsius to produce a micronized powder;
digesting and solubilizing (140) the ground tissue;
neutralizing (145) the solubilized and digested tissue to form an intermediate hydrogel;
lyophilizing (155) the intermediate hydrogel to produce a lyophilized intermediate hydrogel;
cryogenic grinding (160) the lyophilized intermediate hydrogel at a temperature of -20 degrees Celsius or less to produce a powder; and
sterilizing (180) the powder using either an electron beam or gamma radiation.

2. The method of claim 1, wherein grinding (160) the lyophilized intermediate hydrogel includes production of a powder of less than 900 µm, 700 µm, 500 µm, 400 µm, 300 µm, 200 µm or 100 µm (D50).

3. The method of any of the preceding claims, further comprising, after grinding (160) the lyophilized intermediate hydrogel, a sterilization (180) performed using an irradiation dose between 15 and 25 kGy.

4. The method of any of the preceding claims, wherein the micronizing or grinding step includes:
cryogenic grinding in an oscillating grinding system including a grinding tank, wherein the grinding tank is maintained during cryogenic grinding at a temperature equal to or less than -40 Degrees Celsius;
use of an ethanol as a refrigerant solution configured to cool the tissue during grinding; and
grinding at a speed between 1000 and 2000 rpm/min using grinding balls having diameters of 1, 2, 3, 5, 8 and/or 10 millimeter.

5. The method of any of the preceding claims, wherein solubilizing (140) the tissue includes using an alkaline solution or is performed at a neutral pH using collagenases.

6. A therapeutic powder manufactured according to the method of any of the preceding claims.

7. The therapeutic powder of claim 6, wherein the powder includes a particle size (D50) less than 900 µm, 700 µm, 500 µm, 400 µm, 300 µm, 200 µm or 100 µm;
and wherein the ground powder is configured to form a gel, a liquid or a paste upon rehydration.

8. The therapeutic powder of claim 6 or 7, wherein the powder further comprises pharmaceutical agents, therapeutic agents, or preservatives.

## Patentansprüche

1. Ein Verfahren zur Herstellung eines therapeutischen Materials, wobei das Verfahren aufweist:
Erhalten (110) von Schweineplazentagewebe, das vor dem Einfrieren mindestens 2 Stunden lang in einer antibakteriellen oder antiviralen Lösung gewaschen wurde;
Dezellularisieren (125) des Gewebes;
Gefriertrocknen (130) des dezellularisierten Gewebes;
kryogenes Mahlen (135) des gefriergetrockneten Gewebes bei einer niedrigen Temperatur von weniger als 0 Grad Celsius, um ein mikronisiertes Pulver herzustellen;
Aufschließen und Solubilisieren (140) des gemahlenen Gewebes;
Neutralisieren (145) des solubilisierten und aufgeschlossenen Gewebes, um ein Zwischenhydrogel zu bilden;
Gefriertrocknen (155) des Zwischenhydrogels, um ein gefriergetrocknetes Zwischenhydrogel herzustellen;
kryogenes Mahlen (160) des gefriergetrockneten Zwischenhydrogels bei einer Temperatur von -20 Grad Celsius oder weniger, um ein Pulver herzustellen; und
Sterilisieren (180) des Pulvers unter Verwendung entweder eines Elektronenstrahls oder von Gammastrahlung.

2. Das Verfahren nach Anspruch 1, wobei das Mahlen (160) des gefriergetrockneten Zwischenhydrogel die Herstellung eines Pulvers mit einer Partikelgröße von weniger als 900 µm, 700 µm, 500 µm, 400 µm, 300 µm, 200 µm oder 100 µm (D50) umfasst.

3. Das Verfahren nach einem der vorhergehenden Ansprüche, ferner aufweisen eine Sterilisation (180), die nach dem Mahlen (160) des gefriergetrockneten Zwischenhydrogel unter Verwendung einer Bestrahlungsdosis zwischen 15 und 25 kGy durchgeführt wird.

4. Das Verfahren nach einem der vorhergehenden Ansprüche, wobei der Mikronisierungs- oder Mahlschritt umfasst:
kryogenes Mahlen in einem oszillierenden Mahlsystem, das einen Mahlbehälter umfasst, wobei der Mahlbehälter während des kryogenen Mahlens auf einer Temperatur von -40 Grad Celsius oder darunter gehalten wird;
Verwendung von Ethanol als Kühlmittel, das dazu dient, das Gewebe während des Mahlens zu kühlen; und
Mahlen mit einer Geschwindigkeit zwischen 1000 und 2000 U/min unter Verwendung von Mahlkugeln mit Durchmessern von 1, 2, 3, 5, 8 und/oder 10 Millimetern.

5. Das Verfahren nach einem der vorhergehenden Ansprüche, wobei das Solubilisieren (140) des Gewebes Verwenden einer alkalischen Lösung umfasst oder bei einem neutralen pH-Wert unter Verwendung von Kollagenasen durchgeführt wird.

6. Ein therapeutisches Pulver, das nach dem Verfahren eines der vorhergehenden Ansprüche hergestellt wurde.

7. Das therapeutische Pulver nach Anspruch 6, wobei das Pulver eine Partikelgröße (D50) von weniger als 900 µm, 700 µm, 500 µm, 400 µm, 300 µm, 200 µm oder 100 µm umfasst;
und wobei das gemahlene Pulver derart konfiguriert ist, dass es bei Rehydrierung ein Gel, eine Flüssigkeit oder eine Paste bildet.

8. Das therapeutische Pulver nach Anspruch 6 oder 7, wobei das Pulver ferner pharmazeutische Wirkstoffe, therapeutische Wirkstoffe oder Konservierungsmittel aufweist.

## Revendications

1. Procédé de fabrication d'un matériau thérapeutique, le procédé comprenant :
l'obtention (110) de tissu placentaire porcin ayant été lavé pendant au moins 2 heures dans une solution antibactérienne ou antivirale avant congélation ;
la décellularisation (125) du tissu ;
la lyophilisation (130) du tissu décellularisé ;
le broyage cryogénique (135) du tissu lyophilisé à basse température de moins de 0 degrés Celsius pour produire une poudre micronisée ;
la digestion et la solubilisation (140) du tissu broyé ;
la neutralisation (145) du tissu solubilisé et digéré pour former un hydrogel intermédiaire ;
la lyophilisation (155) de l'hydrogel intermédiaire pour obtenir un hydrogel intermédiaire lyophilisé ;
broyage cryogénique (160) de l'hydrogel intermédiaire lyophilisé à une température inférieure ou égale à -20 degrés Celsius pour produire une poudre ; et
stérilisation (180) de la poudre en utilisant soit un faisceau d'électrons, soit un rayonnement gamma.

2. Procédé selon la revendication 1, dans lequel le broyage (160) de l'hydrogel intermédiaire lyophilisé comprend la production d'une poudre inférieure à 900 µm, 700 µm, 500 µm, 400 µm, 300 µm, 200 µm ou 100 µm (D50).

3. Procédé selon l'une quelconque des revendications précédentes, comprenant en outre, après le broyage (160) de l'hydrogel intermédiaire lyophilisé, une stérilisation (180) réalisée en utilisant une dose d'irradiation comprise entre 15 et 25 kGy.

4. Procédé selon l'une quelconque des revendications précédentes, dans lequel l'étape de micronisation ou de broyage comprend :
le broyage cryogénique dans un système de broyage oscillant comprenant un réservoir de broyage, dans lequel le réservoir de broyage est maintenu pendant le broyage cryogénique à une température égale ou inférieure à -40 degrés Celsius;
l'utilisation d'un éthanol comme solution réfrigérante configurée pour refroidir le tissu pendant le broyage ; et le broyage à une vitesse comprise entre 1000 et 2000 tr/min en utilisant des billes de broyage d'un diamètre de 1, 2, 3, 5, 8 et/ou 10 millimètres.

5. Procédé selon l'une quelconque des revendications précédentes, dans lequel la solubilisation (140) du tissu comprend l'utilisation d'une solution alcaline ou est réalisée à un pH neutre au moyen de collagénases.

6. Poudre thérapeutique fabriquée selon le procédé de l'une quelconque des revendications précédentes.

7. Poudre thérapeutique selon la revendication 6, dans laquelle la poudre comprend une taille de particule (D50) inférieure à 900 µm, 700 µm, 500 µm, 400 µm, 300 µm, 200 µm ou 100 µm ; et dans lequel la poudre broyée est configurée pour former un gel, un liquide ou une pâte lors de la réhydratation.

8. Poudre thérapeutique selon la revendication 6 ou 7, dans laquelle la poudre comprend en outre des agents pharmaceutiques, des agents thérapeutiques ou des conservateurs.
